(19) 

Europäisches Patentamt
European Patent Office
Office européen des brevets

(11) **EP 4 410 281 A1**

(12) **EUROPEAN PATENT APPLICATION**

(43) Date of publication:
**07.08.2024 Bulletin 2024/32**

(21) Application number: **23382105.7**

(22) Date of filing: **06.02.2023**

(51) International Patent Classification (IPC):
*A61K 9/51* (2006.01)    *A61K 9/00* (2006.01)
*A61K 36/738* (2006.01)    *A61K 31/352* (2006.01)
*A61P 27/00* (2006.01)    *A61P 27/06* (2006.01)
*A61P 27/12* (2006.01)    *A61P 31/04* (2006.01)
*A61P 31/12* (2006.01)    *A61P 35/00* (2006.01)

(52) Cooperative Patent Classification (CPC):
(C-Sets available)
**A61K 9/0048; A61K 9/5123; A61K 31/352;
A61K 36/738; A61P 27/00; A61P 27/06;
A61P 27/12; A61P 31/04; A61P 31/12; A61P 35/00**

(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Universitat de Barcelona
08028 Barcelona (ES)**

(72) Inventors:
• **SÁNCHEZ LÓPEZ, Elena
08028 Barcelona (ES)**
• **GARCÍA LÓPEZ, María Luisa
08028 Barcelona (ES)**
• **BONILLA VIDAL, Lorena
08028 Barcelona (ES)**
• **ESPINA GARCÍA, Marta
08028 Barcelona (ES)**

(74) Representative: **Hoffmann Eitle
Hoffmann Eitle S.L.U.
Paseo de la Castellana 140, 3a planta
Edificio LIMA
28046 Madrid (ES)**

(54) **LIPID NANOPARTICLES FOR THE TREATMENT OF OCULAR DISEASES**

(57)    The present invention relates to a composition comprising lipid nanoparticles comprising at least one liquid lipid with anti-inflammatory properties, such as for example rosehip oil, at least one solid lipid, at least one cationic surfactant, optionally at least one non-cationic surfactant and optionally at least one active ingredient, as well as their use in a method of treating ocular diseases, such as for example dry eye disease.

FIG.7

Schirmer test

**EP 4 410 281 A1**

**(Cont. next page)**

(52) Cooperative Patent Classification (CPC): (Cont.)

C-Sets
**A61K 31/352, A61K 2300/00;**
**A61K 36/738, A61K 2300/00**

**Description**

**TECHNICAL FIELD**

The present invention relates to a composition comprising lipid nanoparticles comprising at least one liquid lipid with anti-inflammatory properties, such as for example rosehip oil, at least one solid lipid, at least one cationic surfactant and optionally, at least a non-cationic surfactant, and optionally at least one active ingredient, as well as their use in a method of treating ocular diseases, such as for example dry eye disease.

**BACKGROUND OF THE INVENTION**

Dry eye disease (DED) is a condition frequently encountered in ophthalmology practice worldwide. DED is a multifactorial disease of the ocular surface characterized by a loss of homeostasis of the tear film, and accompanied by ocular symptoms, in which tear film instability and hyperosmolarity, ocular surface inflammation and damage, and neurosensory abnormalities play etiological roles. The prevalence of DED in the population ranges from 5 - 34 % depending on the country [1]. Topical administration is the preferred route to treat DED because it is painless and easy to handle. Artificial tears in the form of eyedrops, gel or ointment are used to lubricate dry eyes maintaining moisture of the eye's surface and often constitute the first line of therapy. They instantly relieve symptoms by lowering osmolarity and diluting inflammatory markers. However, artificial tears have no anti-inflammatory properties and do not deal with the fundamental pathogenesis of the disease. Moreover, usual treatments for ocular inflammation comprise corticosteroids and non-steroidal anti-inflammatory drugs (NSAIDs), but its prolonged use involves severe side effects, such as increasing ocular pressure and cataract formation [2,3].

Plant oils have been used for a variety of purposes with their integration into foods, cosmetics, and pharmaceutical products. Specifically, the use of plant oils for topical skin applications as well as the therapeutic benefits of these plant oils according to their anti-inflammatory and antioxidant effects on the skin have been described. One of the plant oils used for such applications is rosehip oil (*R.canina*).

Apigenin (APG) is a natural flavonoid that has been used in the form of plant extract for the treatment of several disorders and inflammatory conditions. Of all the flavonoids, APG is one of the most widely distributed in plants, and one of the most studied phenolics. APG is present in significant amount in its glycosylated form in vegetables, fruits, herbs and plant-based beverages. Based on preclinical and clinical data, it has been suggested that APG is a potent therapeutic agent to overcome diseases, such as inflammatory diseases, bacterial, viral, and parasitic infections, autoimmune disorders, diabetes, hypertension, hypercholesterolemia, and various types of cancers [4,5].Several clinical trials have been carried out for the use of APG as a dietary supplement. In clinical trial NCT04114916, the reduction of cardiovascular risk in healthy subjects has been evaluated [6]. In clinical trial NCT01286324 APG, as a natural part of a chamomile extract, was studied as a dietary supplement for the treatment of chronic primary insomnia. It was concluded that the extract could provide modest benefits of daytime functioning and mixed benefits on sleep diary measures relative to placebo in adults with chronic primary insomnia [7]. Furthermore, APG itself is currently commercialized as a dietary supplement in Spain in the form of capsules containing 50 mg APG for improving prostate health, decrease glucose levels, and maintain the function of the nervous system [8,9].

Whilst commercialized eye drops containing chamomile extract for the enhancement of ocular discomfort, such as irritation, tired eyes, and itchiness are on the market [10,11], APG itself has to date not been suggested or approved as a therapy for ocular diseases, such as for example DED. This may partially be due to the disadvantages of APG for topical treatments caused among other by its low solubility and low bioavailability [12].

In the recent years new delivery systems, such as lipid nanoparticles, have been developed which provide multiple advantages, such as low in vivo toxicity, good long-term stability, economic and solvent-free production techniques, easy production at large scale, possibility to be autoclaved or sterilized, increased kinetic stability compared to liposomes and niosomes [13]. Moreover, the last generation of lipid nanoparticles, nanostructured lipid carriers (NLC), represent a topical delivery system with improved stability in comparison to the solid lipid nanoparticles (SLN).

As mentioned above topical administration is a preferred route to treat DED. The currently available remedies using artificial tears have no anti-inflammatory properties and do not deal with the fundamental pathogenesis of the disease. Available treatment options for ocular inflammation in general include corticosteroids and NSAIDs which after prolonged use can be the cause of severe side effects. Another drawback of currently available topical applications is the generally fast release of the active ingredient(s). Furthermore, topical applications into the eye require that the active ingredient is able to permeate through the corneal barrier to reach its destination.

To date, no lipid nanoparticles containing liquid lipids have been described that are suitable for topical use in the ocular system and that can provide for benefits/treatment for ocular inflammatory diseases, such as DED. The inventors have therefore set out to develop lipid nanoparticles with intrinsic activity that are suitable for such applications and that provide biocompatibility, capability to reverse disease symptoms as well as anti-inflammatory efficacy.

In addition, the inventors have set out to provide a topical delivery system that is suitable for the application of APG overcoming its low solubility and low bioavailability, and that furthermore provides for sustained drug release and corneal permeability which is essential for improving the pharmacokinetic and pharmacodynamic profile of APG.

## BRIEF DESCRIPTION OF THE FIGURES

**Fig.1**: Scheme of the evaluation of the fluorescein staining on the ocular surface. 9: maximum score; 0: minimum score.

**Fig.2:** Scheme of the evaluation of bengal rose staining on the ocular surface. 9: maximum score; 0: minimum score.

**Fig.3:** Design of Experiments (DoE) surface response of Loaded Lipid Nanoparticles. (A) Concentration of APG (%) and glyceryl dibehenate + rosehip oil (%) influence on $Z_{av}$ (nm). (B) Concentration of Polysorbate 80 (%) and glyceryl dibehenate + rosehip oil (%) influence on PDI. (C) Concentration of Polysorbate 80 (%) and glyceryl dibehenate + rosehip oil (%) influence on ZP (mV). (D) Concentration of APG (%) and glyceryl dibehenate + rosehip oil (%) influence on EE (%).

**Fig.4:** Physicochemical characterization of optimized Loaded Lipid Nanoparticles, empty lipid nanoparticles and apigenin. (A) Transmission electron microscopy (TEM) image of loaded lipid nanoparticles (scale bar 100 nm). (B) Differential scanning calorimetry (DSC) curves. (C) X-ray diffraction (XRD) patterns. (D) Fourier-transformed infrared (FTIR) analysis.

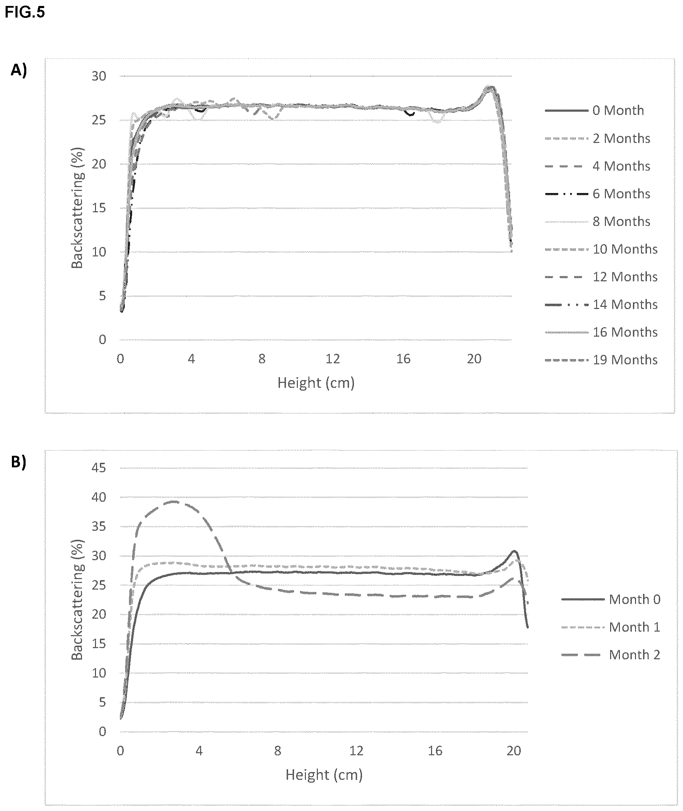

**Fig.5:** Backscattering profiles of Loaded Lipid Nanoparticles stored at: (A) 4 °C and, (B) 25 °C.

**Fig.6:** Biopharmaceutical behavior. In vitro APG release from Loaded Lipid nanoparticles against free-APG (adjusted to two phase decay and plateau followed by one phase decay respectively) and pharmacokinetic parameters Loaded Lipid Nanoparticles applied to a one-phase association.

**Fig.7:** Results of the Schirmer test on New Zealand rabbits after dry eye disease induction with statistically significant differences (* $p < 0.05$, ** $p < 0.01$, *** $p < 0.001$; **** $p < 0.0001$).

**Fig.8:** Fluorescein staining of the ocular surface of New Zealand rabbits after dry eye disease induction. Results of the fluorescein staining with statistically significant differences (* $p < 0.05$, ** $p < 0.01$, and *** $p < 0.001$) of: Dry eye, Lipid Nanoparticles, Loaded Lipid Nanoparticles, Free APG, Commercial solution.

**Fig.9:** Bengal rose staining of the ocular surface of New Zealand rabbits after dry eye disease induction. Results of the Bengal rose staining with statistically significant (* $p < 0.05$) of: Dry eye, Lipid Nanoparticles, Loaded Lipid Nanoparticles, Free APG, Commercial solution.

**Fig.10:** Ocular inflammation prevention results on New Zealand rabbits. Values are expressed as mean $\pm$ SD; * $p < 0.05$, ** $p < 0.01$ and *** $p < 0.001$ and **** $p < 0.0001$ significantly different ocular inflammation score.

**Fig.11:** Ocular inflammation treatment results on New Zealand rabbits. Values are expressed as mean $\pm$ SD; * $p < 0.05$, ** $p < 0.01$ and *** $p < 0.001$ and **** $p < 0.0001$ significantly different ocular inflammation score.

## BRIEF DESCRIPTION OF THE INVENTION

The problem addressed by the present invention is to provide a topical ocular nanoparticulated lipid system that provides biocompatibility, the capability to treat ocular disease or reverse their symptoms, treat and/or prevent ocular inflammation and the capacity to act as drug delivery carrier. A further problem addressed is the provision of a topical delivery system for APG that overcomes its disadvantages, such as its low solubility and bioavailability and that provides for sustained drug release and corneal permeability and thus improved pharmacokinetic and pharmacodynamic profiles of APG.

## SUMMARY OF THE INVENTION

In one aspect the present invention relates to a composition comprising lipid nanoparticles, said lipid nanoparticles comprising

a. at least one liquid lipid with anti-inflammatory properties;
b. at least one solid lipid;
c. at least one cationic surfactant
d. optionally, at least one non-cationic surfactant;
e. optionally, at least one active ingredient.

In one embodiment of the composition of present invention the said liquid lipid with anti-inflammatory properties is selected from the group consisting of rosehip oil, tea tree oil, lavender oil, linoleic acid, stearic acid, palmitic acid, castor oil, safflower oil, melon seed oil, salicornia oil, evening primrose oil, poppyseed oil, grape seed oil, prickly pear oil, artichoke oil, hemp oil, wheat germ oil, cottonseed oil, corn oil, walnut oil, soybean oil, sesame oil, rice bran oil, argan oil, pistachio oil, peach oil, almond oil, canola oil, avocado oil, flaxseed oil, sunflower oil, peanut oil, palm oil, olive oil, macadamia oil, coconut oil, rosemary oil, lavender oil, origanum vulgare oil, thyme oil, mint oil, eucalyptus oil, ginger oil, cuminum cyminum l. oil, turmeric oil, clove oil, oleic acid, oregano oil, rose oil, fennel oil, bergamot oil, chamomile oil, helichrysum oil, patchouli oil, frankincense oil, copaiba oil, peppermint oil, black pepper oil, sweet marjoram oil, basil oil, clove oil, clary sage oil, lemongrass oil, geranium oil, wintergreen oil, cannabis oil, cannabidiol, spruce oil, niaouli oil, cardamom oil, pine tree oil, fir tree oil, juniper tree oil, verbena oil, marjoram oil, katafray oil, bitter orange oil, hypericum oil, arnica oil, coriander oil, mustard oil, perilla seed oil, centella asiatica oil, calendula oil, laurel oil, camphor oil, cinnamon oil, oatmeal oil, docosahexaenoic acid, eicosapentaenoic acid, dandelion oil, krill oil, electrophorus electricus oil, potamotrygon motoro oil, boa constrictor oil, chelonoidis denticulate oil, melanosuchus niger oil, inia geoffrensis oil, horse oil, anchovy oil, prunus seed oil, tropidurus hispidus oil, emu oil, maqian fruits essential oil, fructus alpinia oil, cinnamomum cassia essential oil, angelica sinensis oil, gynura procumbens oil, spirulina oil, citrus limetta oil, citrus aurantium oil, atractylodes macrocephala oil, artemisia argyi oil, gynura procumbens oil, acorus gramineusand oil, algal oil, fish oil, zanthoxylum coreanum nakai oil, cod liver oil, perna canaliculus oil, chia seed oil, or combinations thereof.

In one embodiment of present invention the said liquid lipid with anti-inflammatory properties is selected from derivatives of the above listed liquid lipids.

In a preferred embodiment the said liquid lipid with anti-inflammatory properties is rosehip oil.

In one embodiment of the composition of the present invention the solid lipid is selected from monoglycerides, diglycerides, triglycerides, cholesterols, steroids, fatty alcohols, glycerol esters glyceryl tridecanoate, glycerol trilaurate, glyceryl trimyristate, glyceryl tripalmitate, glyceryl tristearate, hydrogenated coco-glycerides, hard fat types, mixtures of triglycerides and/or diglycerides and/or monoglycerides and/or glycerol, acyl glycerols, glyceryl monostearate, glyceryl distearate, glyceryl monooleate, glyceryl dibehenate, glyceryl palmitostearate, waxes, cetyl palmitate, fatty acids, stearic acid, palmitic acid, decanoic acid, behenic acid, glycerol stearate citrate, polyethylene glycol monostearate, cyclic complexes, cyclodextrin para-acyl-calix-arenes, or mixtures thereof.

In a preferred embodiment said solid lipid is glyceryl dibehenate.

In one embodiment of the composition comprising lipid nanoparticles of present invention the cationic surfactant is selected from the group consisting of dimethyl-dioctadecyl-ammonium bromide (DDAB), dioleoyl phosphatidylethanolamine, 1,2-distearyloxy-N,N-dimethyl-3-aminopropane, 1,2 dioleyloxy-N,N-dimethyl-3-aminopropane,1,2-dilinoleyloxy-N,N-dimethyl-3-aminopropane, 1,2-dilinolenyloxy-N,N-dimethyl-3-aminopropane, cetyltrimethylammonium bromide, 3β-[N(N',N'-dimethylaminoethane)-carbamoyl]cholesterol, 1,2-dioleoyl-3-trimethylammonium-propane, 1,2-dimyristoyl-3-trimethylammonium-propane, 1,2-stearoyl-3-trimethylammonium-propane, N-(4-carboxybenzyl)-N,N-dimethyl-2,3-bis(oleoyl-oxy)propan-1-aminium, 1-Palmitoyl-2-oleoyl-sn-glycero-3-phosphoethanolamine, N,N-di-(β-stearoylethyl)-N,N-dimethyl-ammonium chloride, benzalkonium chloride, cetylpyridinium chloride, cetrimide, N-[1-(2,3-dioleoyloxy)propyl]-N,N,N-trimethylammonium chloride, 1,2-dilineoyl-3-dimethylammonium-propane, 1,2-dilinoleyloxy-3-N,N-dimethylaminopropane, 1,2-dilinoleyloxy- keto-N,N-dimethyl-3-aminopropane, 1,2-dilinoleyl-4-(2- dimethylaminoethyl)-[1,3]-dioxolane(3-o-[2"-(meth oxypolyethyleneglycol 2000) succinoyl]-1,2-dimyristoyl-sn-glycol, R-3-[((ω-methoxy-poly(ethyleneglycol)2000)carbamoyl]-1,2-dimyristyloxlpropyl-3-amine, cetyltrimethylammo-nium bromide, octadecylamine, 1-oleoyl-rac-glycerol, octadecyl quaternized carboxymethyl chitosan, hexadecyl trimethyl ammonium bromide.

In a preferred embodiment of the composition of present invention the cationic surfactant is dimethyldioctadecylammonium bromide (DDAB).

In a further embodiment of the composition of present invention the non-cationic surfactant is selected from the group consisting of polysorbate 80, soya lecithin, sodium dodecyl sulphate, polysorbate 20, polysorbate 40, polysorbate 60, PEG-30 glyceryl stearate, cholic acid, phosphatidyl choline, phospholipids with phosphatidylcholine, egg lecithin, poloxamer 188, poloxamer407, poloxamer 184, poloxamer 338, poloxamine 908, tyloxopol, taurocholate sodium salt, taurodeoxycholicacid sodium salt, sodium glycocholate, sodium oleate, cholesteryl hemisuccinate, butanol, sodium cholate, nonionic polyoxyethylene, non-ionic amphiphilic surfactant with an alkyl moiety and an ethylene oxide chain, palmitic acid, stearic acid, mixtures of palmitic and stearic acid, lecithin, polyglycerol 6-distearate, caprylyl/capryl glucoside, coco-glucoside, sucrose palmitate, sucrose stearate, sucrose distearate, tyloxapol, lecithin, cetylpyridinium chloride, sorbitan laurate, polyethylene glycol ether of cetyl or stearyl alcohol, castor oil polyoxyethylene ether, macrogolglycerol ricinoleate, dioctyl sodium sulfosuccinate, monooctylphosphoric acid sodium, hexadecyl trimethyl ammonium bromide, polyvinyl alcohol, polyoxyethylene (40) stearate, polyethylene glycol-polypropylene glycol-polyethylene glycol triblock copolymer, olyoxyethylene nonylphenyl ether, hexadecyltrimethylammonium bromide, sodium dodecyl sulfate, sodium cholate, stearate sodium hydrolysed polyvinyl alcohol 9000-10000 MW, dioctyl sulfosuccinate, taurocholate, 4-dodecylbenzenesulfonic acid, long chain carboxylic acid, alkyldiphenyloxide disulfonate, or mixtures thereof.

In a preferred embodiment the non-cationic surfactant is polysorbate 80.

In one embodiment the at least one active ingredient is apigenin (APG).

In one embodiment the at least one active ingredient, preferably apigenin, is encapsulated in the lipid nanoparticles.

In a further embodiment said lipid nanoparticles further comprise a coating, preferably wherein said coating comprises hyaluronic acid.

In one preferred embodiment of the composition of present invention said lipid nanoparticles comprise

    a. 0.01 - 30 % (w/v) lipid liquid with anti-inflammatory properties, preferably rosehip oil;
    b. 1 - 50 % (w/v) solid lipid, preferably glyceryl dibehenate;
    c. 0.001 - 10 % (w/v) cationic surfactant, preferably DDAB;
    d. optionally 0.00 - 10 % (w/v) non-cationic surfactant, preferably polysorbate 80;
    e. optionally 0.00 - 10 % (w/v) active ingredient, preferably apigenin.

In one preferred embodiment of the composition of present invention said lipid nanoparticles comprise

    a. 2.5 - 12.5% (w/v) lipid liquid with anti-inflammatory properties, preferably rosehip oil;
    b. 1 - 30 % (w/v) solid lipid, preferably glyceryl dibehenate;
    c. 0.025 - 0.5 % (w/v) cationic surfactant, preferably DDAB;
    d. 1.0 - 5.0% (w/v) non-cationic surfactant, preferably polysorbate 80;
    e. optionally 0.1 - 2.5% (w/v) active ingredient, preferably apigenin.

In one preferred embodiment of the composition of present invention said lipid nanoparticles comprise

    a. 3.0% (w/v) rosehip oil,
    b. 4.5 % (w/v) glyceryl dibehenate,
    c. 0.05% (w/v) DDAB,
    d. 3.5% (w/v) polysorbate 80,
    e. optionally 0.1% (w/v) apigenin.

In one embodiment the lipid nanoparticles further comprise from 0.00001 % - 0.001 % (w/v), preferably 0.0001 % (w/v) hyaluronic acid, more preferably wherein said hyaluronic acid coats the lipid nanoparticles.

In another aspect the present invention relates to a composition comprising lipid nanoparticles as described herein for use in the treatment, amelioration or prevention of ocular diseases, such as ocular inflammation, glaucoma, ocular tumors, bacterial and viral infections of the eye, age-related macular degeneration, cataracts, diabetic retinopathy, or dry eye disease (DED).

In one embodiment the composition is administered topically into the eye.

## DETAILED DESCRIPTION OF THE INVENTION

### DEFINITIONS

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

The terms "comprise" and "comprising" are used in the inclusive, open sense, meaning that additional elements may be included. The term "comprises" also encompasses and may be used interchangeably with the terms "consists of" and "consists essentially of".

## DETAILED DESCRIPTION

Dry eye disease (DED) is a condition frequently encountered in ophthalmology practice worldwide. Topical administration is a preferred route to treat DED, but currently available artificial tears have no anti-inflammatory properties and do not deal with the fundamental pathogenesis of the disease. Usual treatments for ocular inflammation include corticosteroids and non-steroidal anti-inflammatory drugs (NSAIDs) which after prolonged use led to severe side effects. Another drawback of topical applications is the generally fast release of the active ingredient(s). Furthermore, for topical applications into the eye, it needs to be assured that the active ingredient can permeate through the corneal barrier to reach its destination.

The inventors have therefore set out to develop a drug delivery system that can overcome these drawbacks and that provides biocompatibility, anti-inflammatory efficacy as well as capability to reverse ocular disease symptoms, such as DED. A further aim of the invention is to provide an ocular topical delivery system for APG that overcomes its low solubility and bioavailability and that provides for sustained drug release as well as corneal permeability thus improving the pharmacokinetic and pharmacodynamic profile of APG.

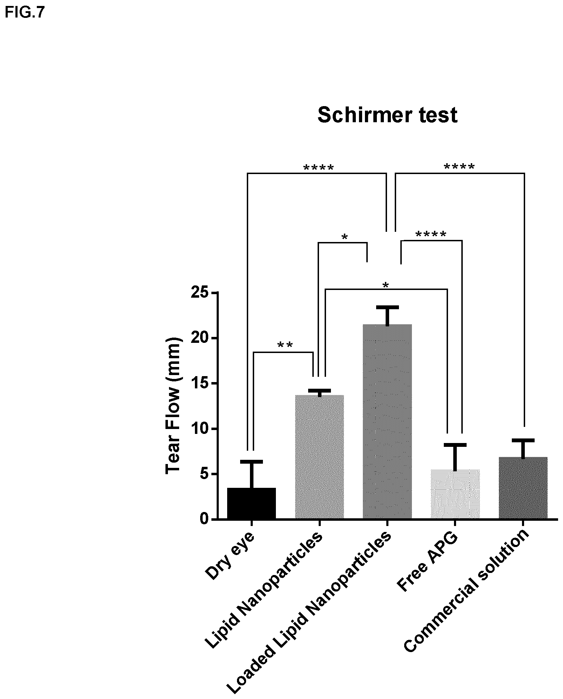

To achieve this the inventors have developed lipid nanoparticles comprising rosehip oil extracted from Rosa canina seeds as a liquid lipid having antioxidant, anti-inflammatory and regenerative properties. Rosehip oil contains essential fatty acids, tocopherols, sterols and phenolics with functional properties (15). The inventors could show that the lipid nanoparticles according to present invention comprising rosehip oil and no further active ingredient, herein named "lipid nanoparticles", already provide for an improved tear flow, could restore the corneal surface and were able to restore the tear film (see Example 7, Figures 7-9).

In one aspect the present invention relates to a composition comprising lipid nanoparticles, said lipid nanoparticles comprising

a. at least one liquid lipid with anti-inflammatory properties;
b. at least one solid lipid;
c. at least one cationic surfactant
d. optionally, at least a non-cationic surfactant;
e. optionally, at least one active ingredient.

In one embodiment the at least one said liquid lipid with anti-inflammatory properties is selected from the group consisting of rosehip oil, tea tree oil, lavender oil, linoleic acid, stearic acid, palmitic acid, castor oil, safflower oil, melon seed oil, salicornia oil, evening primrose oil, poppyseed oil, grape seed oil, prickly pear oil, artichoke oil, hemp oil, wheat germ oil, cottonseed oil, corn oil, walnut oil, soybean oil, sesame oil, rice bran oil, argan oil, pistachio oil, peach oil, almond oil, canola oil, avocado oil, flaxseed oil, sunflower oil, peanut oil, palm oil, olive oil, macadamia oil, coconut oil, rosemary oil, lavender oil, origanum vulgare oil, thyme oil, mint oil, eucalyptus oil, ginger oil, cuminum cyminum I. oil, turmeric oil, clove oil, oleic acid, oregano oil, rose oil, fennel oil, bergamot oil, chamomile oil, helichrysum oil, patchouli oil, frankincense oil, copaiba oil, peppermint oil, black pepper oil, sweet marjoram oil, basil oil, clove oil, clary sage oil, lemongrass oil, geranium oil, wintergreen oil, cannabis oil, cannabidiol, spruce oil, niaouli oil, cardamom oil, pine tree oil, fir tree oil, juniper tree oil, verbena oil, marjoram oil, katafray oil, bitter orange oil, hypericum oil, arnica oil, coriander oil, mustard oil, perilla seed oil, centella asiatica oil, calendula oil, laurel oil, camphor oil, cinnamon oil, oatmeal oil, docosahexaenoic acid, eicosapentaenoic acid, dandelion oil, krill oil, electrophorus electricus oil, potamotrygon motoro oil, boa constrictor oil, chelonoidis denticulate oil, melanosuchus niger oil, inia geoffrensis oil, horse oil, anchovy oil, prunus seed oil, tropidurus hispidus oil, emu oil, maqian fruits essential oil, fructus alpinia oil, cinnamomum cassia essential oil, angelica sinensis oil, gynura procumbens oil, spirulina oil, citrus limetta oil, citrus aurantium oil, atractylodes macrocephala oil, artemisia argyi oil, gynura procumbens oil, acorus gramineusand oil, algal oil, fish oil, zanthoxylum coreanum nakai oil, cod liver oil, perna canaliculus oil, chia seed oil, or combinations thereof.

The above listed liquid lipids are known to contain anti-inflammatory properties and are thus suitable for use in the compositions of present invention. Anti-inflammatory properties herein refer to the ability of the liquid lipid to reduce inflammation and/or redness, and/or pain and/or swelling of a tissue.

The preferred liquid lipid is rosehip oil. The lipid nanoparticles of present invention can comprise from 0.01 - 30.0% (w/v), from 0.1 - 30.0% (w/v), from 0.5 - 25.0% (w/v), from 1.0 - 20.0% (w/v), from 1.5 - 15.0% (w/v), from 2.0 - 14.0% (w/v), from 2.5 - 12.5% (w/v), from 2.5 - 10.0% (w/v), from 2.5 - 9.0% (w/v), from 2.5 - 8.0% (w/v), from 2.5 - 7.0% (w/v), from 2.7 - 6.0% (w/v), from 2.8 - 5.0% (w/v), from 2.9 - 4.0% (w/v) rosehip oil. Preferred is an amount of from 2.5 - 12.5% (w/v), more preferred an amount from 2.9 - 4.0% (w/v), most preferred 3.0% (w/v).

In one embodiment of the composition of the present invention the solid lipid is selected from monoglycerides, diglycerides, triglycerides, cholesterols, steroids, fatty alcohols, glycerol esters glyceryl tridecanoate, glycerol trilaurate, glyceryl trimyristate, glyceryl tripalmitate, glyceryl tristearate, hydrogenated coco-glycerides, hard fat types, mixtures of triglycerides and/or diglycerides and/or monoglycerides and/or glycerol, acyl glycerols, glyceryl monostearate, glyceryl distearate, glyceryl monooleate, glyceryl dibehenate, glyceryl palmitostearate, waxes, cetyl palmitate, fatty acids, stearic acid, palmitic acid, decanoic acid, behenic acid, glycerol stearate citrate, polyethylene glycol monostearate, cyclic complexes, cyclodextrin para-acyl-calix-arenes, or mixtures thereof.

The solid lipid can be present in the lipid nanoparticles of present invention from 1.0 - 50 % (w/v), from 1.0 - 40 % (w/v), from 1.0 - 30 % (w/v), from 1.0 - 20 % (w/v), from 1.0 - 15 % (w/v), from 1.0 - 10 % (w/v), from 1.5 - 9.5 % (w/v), from 2.0 - 9.0 % (w/v), from 2.5 - 8.5 % (w/v), from 3.0 - 8.0 % (w/v), from 3.2 - 7.5 % (w/v), from 3.5 - 7.0 % (w/v), from 3.7 - 6.5 % (w/v), from 4.0 - 6.0% (w/v), from 4.1 - 5.5 % (w/v), from 4.2 - 5.0 % (w/v), from 4.3 - 4.9 % (w/v), from 4.4 - 4.8 % (w/v).

The lipid nanoparticles comprise at least at least one cationic surfactant. The cationic surfactant provides an increased bioavailability of the formulation when administered onto the ocular mucosa. Cationic surfactants promote electrostatic interactions between the surface of the cationic particles and the anionic ocular mucosa, with a considerable improvement of the drug residence time [14].

In one embodiment of the composition of present invention the cationic surfactant is selected from the group consisting of dimethyldioctadecylammonium bromide (DDAB), dioleoyl phosphatidylethanolamine, 1,2-distearyloxy-N,N-dimethyl-3-aminopropane, 1,2 dioleyl-oxy-*N*,*N*-dimethyl-3-aminopropane,1,2-dilinoleyloxy-N,N-dimethyl-3-aminopropane, 1,2 - dilinolen-yloxy-N,N-dimethyl-3- aminopropane, cetyltri-methylammonium bromide, 3R-[N(N',N'-dimethylaminoethane)-carbamoyl]cholesterol, 1,2-dioleoyl-3-trimethylammo-nium-propane, 1,2-dimyristoyl-3-trimethylammonium-propane, 1,2-stearoyl-3-tri-methylammonium-propane, N-(4-carboxybenzyl)-N,N-dimethyl-2,3-bis(oleoyloxy) propan-1-aminium, 1-Palmitoyl-2-oleoyl-sn-glycero-3-phosphoethanolamine, N,N-di-(β-stearoylethyl)-N,N-dimethyl-ammonium chloride, benzalkonium chloride, cetylpyridinium chloride, cetrimide, N-[1-(2,3-dioleoyloxy)propyl]-N,N,N-trimethylammonium chloride, 1,2-dilineoyl-3-dimethylammonium-propane, 1,2-dilinoleyloxy-3-N,N-dimethylaminopropane, 1,2-dilinoleyloxy- keto-N,N-dimethyl-3-aminopropane, 1,2-dilinoleyl-4-(2- dimethylaminoethyl)-[1,3]-dioxolane(3-o-[2"-(meth oxypolyethyleneglycol 2000) succinoyl]-1,2-dimyristoyl-sn-glycol, R-3-[(ω-methoxy-poly(ethyleneglycol)2000) carbamoyl]-1,2-dimyristyloxlpropyl-3-amine, cetyltri-methylammonium bromide, octadecylamine, 1-oleoyl-rac-glycerol, octadecyl quaternized carboxymethyl chitosan, hexadecyl trimethyl ammonium bromide.

A preferred cationic surfactant according to present invention is dimethyl-dioctadecyl-ammonium bromide (DDAB). DDAB is a commercially available double chain cationic surfactant that is for example used in the prior art for preparation of lipid bilayer-protected gold nanoparticles (AuNPs) or for delivery systems into mammalian cells, such as RNAi delivery.

The lipid nanoparticles of present invention can comprise from 0.001 - 10 % (w/v), from 0.0015 - 5 % (w/v), from 0.010 - 4 % (w/v), from 0.015 - 2 % (w/v), from 0.020 - 1 % (w/v), from 0.021 - 0.9 % (w/v), from 0.022 - 0.8 % (w/v), from 0.023 - 0.7 % (w/v), from 0.024 - 0.6 % (w/v), from 0.025 - 0.5 % (w/v), from 0.030 - 0.4 % (w/v), from 0.035 - 0.3 % (w/v), from 0.040 - 0.2 % (w/v), from 0.045 - 0.1 % (w/v) of a cationic surfactant.

In previous studies for ocular delivery DDAB had not shown any toxicity at a concentration of 0.5% (18). As such, in one preferred embodiment the lipid nanoparticles of present invention comprise 0.05% (w/v) of DDAB.

The lipid nanoparticles further comprise at least one further surfactant that is not a cationic surfactant, herein referred to also as "non-cationic surfactant". Surfactant type and concentration play an important role in designing lipid nanoparticles. Lipid nanoparticles are stabilized by different types of surfactants which are efficiently adsorbed onto particles' surfaces reducing the interfacial tension. Either a sole surfactant or a mixture of hydrophilic and lipophilic surfactants can be used for the preparation. A blend can provide improved physical stability and functional properties to the lipid nanoparticles.

In one embodiment of the composition of present invention the non-cationic surfactant is selected from the group consisting of polysorbate 80, soya lecithin, sodium dodecyl sulphate, polysorbate 20, polysorbate 40, polysorbate 60, PEG-30 glyceryl stearate, cholic acid, phosphatidyl choline, phospholipids with phosphatidycholine, egg lecithin, poloxamer 188, poloxamer407, poloxamer 184, poloxamer 338, poloxamine 908, tyloxopol, taurocholate sodium salt, taurodeoxycholicacid sodium salt, sodium glycocholate, sodium oleate, cholesteryl hemisuccinate, butanol, sodium cholate, nonionic polyoxyethylene, non-ionic amphiphilic surfactant with an alkyl moiety and an ethylene oxide chain, palmitic acid, stearic acid, mixtures of palmitic and stearic acid, lecithin, polyglycerol 6-distearate, caprylyl/capryl glucoside, coco-glucoside, sucrose palmitate, sucrose stearate, sucrose distearate, tyloxapol, lecithin, cetylpyridinium chloride, sorbitan laurate, polyethylene glycol ether of cetyl or stearyl alcohol, castor oil polyoxyethylene ether, macrogolglycerol ricinoleate, dioctyl sodium sulfosuccinate, monooctylphosphoric acid sodium, hexadecyl trimethyl ammonium bromide, polyvinyl alcohol, polyoxyethylene (40) stearate, polyethylene glycol-polypropylene glycol-polyethylene glycol triblock copolymer, olyoxyethylene nonylphenyl ether, hexadecyltrimethylammonium bromide, sodium dodecyl sulfate, sodium cholate, stearate sodium hydrolysed polyvinyl alcohol 9000-10000 MW, dioctyl sulfosuccinate, taurocholate, 4-dodecylbenzenesulfonic acid, long chain carboxylic acid, alkyldiphenyloxide disulfonate, or mixtures thereof.

The concentration of the non-cationic surfactant particularly influences the particle size of the lipid nanoparticles. Generally, the higher the non-cationic surfactant concentration, the smaller the particle sizes will be. The non-cationic surfactant can be present in the lipid nanoparticles from 0.01 - 10% (w/v), from 0.1 - 9.5% (w/v), from 0.2 - 9.0% (w/v), from 0.3 - 8.5% (w/v), from 0.4 - 8.0% (w/v), from 0.5 - 7.5% (w/v), from 0.6 - 7.0% (w/v), from 0.7 - 6.5% (w/v), from 0.8 - 6.0% (w/v), from 0.9 - 5.5% (w/v), from 1.0 - 5.0% (w/v), from 1.5 - 4.5% (w/v), from 2.0 - 4.0% (w/v), from 2.5 - 3.5% (w/v). For clarity, the ranges disclosed refer to the amount of non-cationic surfactant in the lipid nanoparticles, thus not including the amount of cationic surfactant. The amount of cationic surfactant is specified further above.

The lipid nanoparticles of present invention can further comprise at least one active ingredient. In one embodiment the at least one active ingredient is selected from the group consisting of flavonoids, resveratrol (3,5,4'-trihydroxy-trans-stilbene) and curcumin. Preferred active ingredients are flavonoids due to their antioxidant and anti-inflammatory properties.

A preferred active ingredient of present invention is apigenin. APG is a natural flavonoid that is contained in various plant extracts, among others chamomile extracts, and has been used as plant extract for the treatment of several disorders and inflammatory conditions. Based on preclinical and clinical data, it has been suggested that APG is a potent therapeutic agent to overcome diseases, such as inflammatory diseases, bacterial, viral, and parasitic infections, autoimmune disorders, diabetes, hypertension, hypercholesterolemia, and various types of cancers [4,5]. Furthermore, APG itself is currently commercialized as a dietary supplement in Spain in the form of capsules containing 50 mg APG for improving prostate health, decrease glucose levels, and maintain the function of the nervous system [8,9].

Whilst commercialized eye drops containing chamomile extract for the enhancement of ocular discomfort, such as irritation, tired eyes, and itchiness are on the market [10,11], APG itself has to date not been suggested or approved as a therapy for ocular diseases, such as for example DED. This may partially be due to the disadvantages of APG for topical treatments caused among other by its low solubility and low bioavailability [12]. The same problem occurs with other flavonoids. To overcome these disadvantages the inventors have encapsulated APG into the lipid nanoparticles as described above. APG encapsulation into biocompatible and biodegradable lipid nanoparticles has been carried out to overcome its compromised stability and increase therapeutic activity and half-life on the ocular surface, granting its prolonged release.

In a further embodiment said lipid nanoparticles further comprise a coating. The coating can comprise carbomers, such as CMC (carboxymethyl cellulose), chitosan or hyaluronic acid. In a preferred embodiment said coating comprises hyaluronic acid. Hyaluronic acid (HA) is one of the most utilised viscosity-building macromolecules in ocular delivery devices. This anionic polysaccharide with ocular mucomimetic properties exhibits the capacity of prolonging the precorneal residence time and reducing surface desiccation [15]. In addition, hyaluronic acid can act as a lubricant and provide for an additional moisturizing effect.

This nanosystem has been proven to be physically stable with a prolonged APG release as well as high corneal permeability, thus improving biopharmaceutical APG behavior. In addition, in vitro and in vivo tests corroborate that the developed formulation is biocompatible without any sign of ocular irritation. As already described above, empty lipid nanoparticles showed an ability to revert DED symptoms due to its composition. When the lipid nanoparticles were loaded with APG ("Loaded Lipid Nanoparticles"), tear secretion improved due to the therapeutic properties of APG. In addition, a complementary anti-inflammatory effect of such Loaded Lipid Nanoparticles is also confirmed (see Example 7, Figures 7-9). Hence, Loaded Lipid Nanoparticles constitute a suitable system for the treatment, amelioration and prevention of ocular inflammatory diseases, such as DED.

In one preferred embodiment of the composition of present invention said lipid nanoparticles comprise

    a. 0.01 - 30 % (w/v) lipid liquid with anti-inflammatory properties, preferably rosehip oil;
    b. 1 - 50 % (w/v) solid lipid, preferably glyceryl dibehenate;
    c. 0.001 - 10 % (w/v) cationic surfactant, preferably DDAB;
    d. optionally 0.00 - 10 % (w/v) non cationic surfactant, preferably polysorbate 80;
    e. optionally 0.00 - 10 % (w/v) active ingredient, preferably apigenin.

In one preferred embodiment of the composition of present invention said lipid nanoparticles comprise

    a. 0.01 - 30 % (w/v) lipid liquid with anti-inflammatory properties, preferably rosehip oil;
    b. 1 - 50 % (w/v) solid lipid, preferably glyceryl dibehenate;
    c. 0.005 - 10 % (w/v) cationic surfactant, preferably DDAB;

    d. 0.01 - 10 % (w/v) non cationic surfactant, preferably polysorbate 80;
    e. optionally 0.01 - 10 % (w/v) active ingredient, preferably apigenin.

In one preferred embodiment of the composition of present invention said lipid nanoparticles comprise

    a. 0.1 - 10 % (w/v) lipid liquid with anti-inflammatory properties, preferably rosehip oil;
    b. 1 - 30 % (w/v) solid lipid, preferably glyceryl dibehenate;
    c. 0.01 - 5 % (w/v) cationic surfactant, preferably DDAB;
    d. 0.5 - 7 % (w/v) non cationic surfactant, preferably polysorbate 80;
    e. optionally 0.01 - 10 % (w/v) active ingredient, preferably apigenin.

In one preferred embodiment of the composition of present invention said lipid nanoparticles comprise

    a. 2.5 - 12.5% (w/v) lipid liquid with anti-inflammatory properties, preferably rosehip oil;
    b. 1 - 30 % (w/v) solid lipid, preferably glyceryl dibehenate;
    c. 0.025 - 0.5 % (w/v) cationic surfactant, preferably DDAB;
    d. 1.0 - 5.0% (w/v) non cationic surfactant, preferably polysorbate 80;
    e. optionally 0.1 - 2.5% (w/v) active ingredient, preferably apigenin.

In one preferred embodiment of the composition of present invention said lipid nanoparticles comprise

    a. 3.0% (w/v) rosehip oil,
    b. 4.5 % (w/v) glyceryl dibehenate,
    c. 0.05% (w/v) DDAB,
    d. 3.5% (w/v) polysorbate 80,
    e. optionally 0.1% (w/v) apigenin.

In one embodiment the lipid nanoparticles further comprise from 0.00001 % - 0.001 % (w/v), preferably 0.0001 % (w/v) hyaluronic acid, more preferably wherein said hyaluronic acid coats the lipid nanoparticles.

In another aspect the present invention relates to a composition comprising lipid nanoparticles as described herein for use in the treatment, amelioration or prevention of ocular diseases, such as ocular inflammation, glaucoma, ocular tumors, bacterial and viral infections of the eye, age-related macular degeneration, cataracts or diabetic retinopathy, Dry Eye Disease. In a preferred embodiment the treatment of, amelioration or prevention of DED is envisaged.

It is finally contemplated that any features described herein can optionally be combined with any of the embodiments of any product, method or medical use of the invention; and any embodiment discussed in this specification can be implemented with respect to any of these. It will be understood that particular embodiments described herein are shown by way of illustration and not as limitations of the invention.

All publications and patent applications are herein incorporated by reference to the same extent as if each individual publication or patent application was specifically and individually indicated to be incorporated by reference.

The following examples serve to illustrate the present invention and should not be construed as limiting the scope thereof.

## EXAMPLES

Materials

APG was obtained from Apollo Scientific (Cheshire, UK). Compritol® 888 ATO (Glyceryl dibehenate) was kindly gifted from Gattefossé (Madrid, Spain). Tween® 80 (Polysorbate 80), Bengal Rose and Fluorescein were purchased to Sigma Aldrich (Madrid, Spain). Rosehip oil was purchased to Acofarma Fórmulas Magistrales (Barcelona, Spain). Dimethyldioactadecylammonium bromide (DDAB) was purchased to TCI Europe (Zwijndrecht, Belgium). Sodium hyaluronate was kindly donated by Bloomage Freda Biopharm (Jinan, China). All others chemical reagents and components used in this research were of analytical grade. A Millipore Milli-Q Plus system was used to obtain purified water.

Example 1: LOADED LIPID NANOPARTICLES PREPARATION AND OPTIMIZATION

The production of Loaded Lipid Nanoparticles was carried out by high-pressure homogenization method (Homogenizer FPG 12800, Stansted, United Kingdom) after generating a primary emulsion with the mixture of components with an Ultraturrax T25 (IKA, Germany) at 8000 rpm for 30 s. The production conditions were 85 °C, three homogenization cycles and 900 bars of pressure [16]. A design of experiments approach (DoE) used in order to optimize formulation parameters. A central composite factorial design (containing 2 replicated centre points, 16 factorial points and 8 axial points) was developed using statistical program Statgraphics Centurion 18® version 18.1.12 software (Virginia, USA). Four independent variables: APG concentration (%), surfactant concentration (%), glyceryl dibehenate + rosehip oil concentration (%) and glyceryl dibehenate concentration contained in the mixture glyceryl dibehenate + rosehip oil (%) were evaluated to determine their influence on the NLC properties. Mean particle diameter size ($Z_{av}$), polydispersity index (PDI), entrapment efficiency (EE) and Zeta potential (ZP) were designated as the dependent variables. Once the optimized formulation was obtained, increasing amounts of a cationic surfactant were added in order to obtain a cationic surface charge. Then, sodium hyaluronate was added to the formulation [16,17].

## 1.1 PHYSICOCHEMICAL PARAMETERS

$Z_{av}$ and PDI were assessed by photon correlation spectroscopy (PCS) with a ZetaSizer Nano ZS (Malvern Instruments, Malvern, UK). ZP was estimated by electrophoretic mobility. For these measurements, samples were diluted with Milli-Q water 1:10 and analysed by triplicate at 25 °C. EE was determined indirectly. Previously to the analysis, the non-loaded drug was separated from NPs by filtration/centrifugation at 14,000 r.p.m. (Mikro 22 Hettich Zentrifugen, Germany) using an Amicon® Ultra 0.5 centrifugal filter device (Amicon Millipore Corporation, Ireland). The encapsulation efficiency (EE) was calculated by the difference between the total amount of drug and the free drug, present in the filtered fraction, using Eq. 1 [18]:

$$EE\ (\%) = \frac{Total\ amount\ of\ APG - Free\ APG}{Total\ amount\ of\ APG} \qquad \text{Eq. 1}$$

The quantification of APG was performed by a modified reverse-phase high-performance liquid chromatography (RP-HPLC). Briefly, samples were quantified using HPLC Waters 2695 (Waters, Massachusetts, USA) separation module and a Kromasil® C18 column (5 μm, 150 × 4.6 mm) with a mobile phase formed by a water phase of 2 % acetic acid and an organic phase of methanol, in a gradient (from 40 % to 60 % of water phase in 5 min and back in next 5 min) at a flow rate of 0.9 mL/min. A diode array detector Waters® 2996 at a wavelength of 300 nm was used to detect the APG and data were processed using Empower 3® Software [16,19].

## 1.2 CHARACTERIZATION OF OPTIMIZED LOADED LIPID NANOPARTICLES

### 1.2.1 Transmission electron microscopy

Transmission electron microscopy (TEM) was used to investigate the morphology of the Loaded Lipid Nanoparticles on a Jeol 1010 (Jeol USA, Dearborn Road, Peabody, MA 01960, USA). Copper grids were activated with UV light and samples were diluted (1:10) and placed on the grid surface to visualize the particles. Samples were previously subjected to negative staining with uranyl acetate (2%) [18].

### 1.2.2 Interaction studies

Interaction studies were carried out with the formulation without HA.

Differential scanning calorimetry (DSC) analysis was performed using a DSC 823e System Mettler-Toledo, Barcelona, Spain. A pan with indium (purity ≥99.95%; Fluka, Switzerland) was used to check the calibration of the calorimetric system. An empty pan served as a reference. The DSC measurements were carried out in the Loaded Lipid Nanoparticles formulations using a heating ramp from 25 to 105 °C at 10 °C/min in a nitrogen atmosphere. Data was evaluated using the Mettler STARe V 9.01 dB software (Mettler-Toledo, Barcelona, Spain) [16,18].

X-ray spectroscopy (XRD) was used to analyse the amorphous or crystalline state of the samples. Samples were sandwiched between 3.6 μm polyester films and exposed to CuKα radiation (45 kV, 40 mA, λ = 1.5418 Å) in the range (2θ) of 2-60° with a step size of 0.026° and a measuring time of 200 s per step [16].

Fourier transform infrared (FTIR) spectra of Loaded Lipid Nanoparticles were obtained using a Thermo Scientific Nicolet iZ10 with an ATR diamond and DTGS detector (Barcelona, Spain).

RESULTS:

a) LOADED LIPID NANOPARTICLES PREPARATION AND OPTIMIZATION

Table 1 shows the effect of independent variables used on dependent variables analysed and the values obtained. $Z_{av}$ values are mostly around 200 nm. The developed Loaded Lipid Nanoparticles exhibit a negative surface charge ZP < -20 mV. In all cases EE was higher than 95 %, thus meaning that APG was completely encapsulated. Most of the formulations have PDI values below 0.3, indicating a homogeneous distribution of nanoparticles [20].

Table 1. Design of experiments and characterization of the different formulations developed.

| | Independent variables | | | | | | | | Dependent variables | | | |
|---|---|---|---|---|---|---|---|---|---|---|---|---|
| | APG (%) | | Liquid Lipid (%) | | Solid Lipid (%) | | Polysorbate 80 (%) | | $Z_{av} \pm SD$ (nm) | $PDI \pm SD$ | $ZP \pm SD$ (mV) | $EE \pm SD$ (%) |
| **Factorial points** | | | | | | | | | | | | |
| A1 | -1 | 1 | -1 | 0.75 | 1 | 4.25 | -1 | 2 | 176.1 ± 2.3 | 0.278 ± 0.009 | -21.7 ± 0.7 | 99.9 ± 0.1 |
| A2 | 1 | 2 | -1 | 1.75 | -1 | 3.25 | 1 | 4 | 221.5 ± 2.5 | 0.313 ± 0.012 | -21.4 ± 0.5 | 96.7 ± 0.1 |
| A3 | 1 | 2 | 1 | 3.50 | -1 | 6.50 | -1 | 2 | 253.9 ± 4.4 | 0.246 ± 0.012 | -22.6 ± 0.3 | 99.9 ± 0.1 |
| A4 | -1 | 1 | 1 | 1.50 | 1 | 8.50 | -1 | 2 | 271.4 ± 5.5 | 0.273 ± 0.008 | -23.0 ± 0.8 | 99.9 ± 0.1 |
| A5 | -1 | 1 | 1 | 3.50 | -1 | 6.50 | -1 | 2 | 243.7 ± 0.7 | 0.236 ± 0.014 | -24.6 ± 0.5 | 99.7 ± 0.3 |
| A6 | -1 | 1 | 1 | 3.50 | -1 | 6.50 | 1 | 4 | 214.7 ± 1.6 | 0.181 ± 0.010 | -18.2 ± 0.9 | 99.9 ± 0.1 |
| A7 | -1 | 1 | -1 | 0.75 | 1 | 4.25 | 1 | 4 | 165.9 ± 1.2 | 0.265 ± 0.012 | -17.6 ± 0.2 | 99.9 ± 0.1 |
| A8 | -1 | 1 | -1 | 1.75 | -1 | 3.25 | -1 | 2 | 161.2 ± 2.3 | 0.255 ± 0.023 | -22.3 ± 0.4 | 95.1 ± 0.2 |
| A9 | 1 | 2 | 1 | 4.50 | -1 | 6.50 | 1 | 4 | 212.2 ± 1.8 | 0.217 ± 0.019 | -19.6 ± 0.2 | 99.9 ± 0.1 |
| A10 | -1 | 1 | 1 | 1.50 | 1 | 8.50 | 1 | 4 | 236.8 ± 2.0 | 0.244 ± 0.017 | -18.4 ± 0.5 | 99.9 ± 0.1 |
| A11 | 1 | 2 | -1 | 0.75 | 1 | 4.25 | 1 | 4 | 191.9 ± 2.7 | 0.318 ± 0.058 | -19.2 ± 0.6 | 99.9 ± 0.1 |
| A12 | -1 | 1 | -1 | 1.75 | -1 | 3.25 | 1 | 4 | 202.7 ± 2.4 | 0.203 ± 0.002 | -18.7 ± 0.2 | 97.7 ± 0.1 |
| A13 | 1 | 2 | 1 | 1.50 | 1 | 8.50 | -1 | 2 | 304.6 ± 5.7 | 0.276 ± 0.007 | -20.1 ± 0.4 | 99.1 ± 0.1 |
| A14 | 1 | 2 | -1 | 1.75 | -1 | 3.25 | -1 | 2 | 188.7 ± 2.3 | 0.374 ± 0.008 | -22.2 ± 0.4 | 99.9 ± 0.1 |
| A15 | 1 | 2 | 1 | 1.50 | 1 | 8.50 | 1 | 4 | 240.5 ± 2.5 | 0.277 ± 0.015 | -18.0 ± 0.4 | 99.6 ± 0.1 |
| A16 | 1 | 2 | -1 | 0.75 | 1 | 4.25 | -1 | 2 | 200.5 ± 2.8 | 0.425 ± 0.015 | -22.4 ± 0.6 | 99.9 ± 0.1 |
| **Axial points** | | | | | | | | | | | | |
| A17 | -2 | 0.5 | 0 | 1.857 | 0 | 5.625 | 0 | 3 | 181.2 ± 1.4 | 0.221 ± 0.029 | -19.5 ± 0.3 | 99.9 ± 0.1 |
| A18 | 2 | 2.5 | 0 | 1.875 | 0 | 5.625 | 0 | 3 | 230.3 ± 2.7 | 0.327 ± 0.007 | -20.2 ± 0.2 | 99.9 ± 0.1 |
| A19 | 0 | 1.5 | -2 | 0.625 | 0 | 1.875 | 0 | 3 | 202.0 ± 3.9 | 0.556 ± 0.015 | -22.3 ± 0.3 | 99.9 ± 0.1 |

(continued)

| Axial points | | | | | | | | | | | | |
|---|---|---|---|---|---|---|---|---|---|---|---|---|
| A20 | 0 | 1.5 | 2 | 3.125 | 0 | 9.375 | 0 | 3 | 280.9 ± 5.7 | 0.259 ± 0.009 | -20.7 ± 0.4 | 99.9 ± 0.1 |
| A21 | 0 | 1.5 | 0 | 4.50 | -2 | 5.50 | 0 | 3 | 187.3 ± 1.3 | 0.195 ± 0.012 | -20.4 ± 0.5 | 99.9 ± 0.1 |
| A22 | 0 | 1.5 | 0 | 0.50 | 2 | 9.50 | 0 | 3 | 202.5 ± 3.3 | 0.331 ± 0.026 | -20.7 ± 0.2 | 99.9 ± 0.1 |
| A23 | 0 | 1.5 | 0 | 2.50 | 0 | 7.50 | -2 | 1 | 271.0 ± 4.3 | 0.231 ± 0.020 | -24.1 ± 0.7 | 99.9 ± 0.1 |
| A24 | 0 | 1.5 | 0 | 2.50 | 0 | 7.50 | 2 | 5 | 239.1 ± 1.6 | 0.182 ± 0.027 | -17.2 ± 0.8 | 98.1 ± 0.3 |
| Central points | | | | | | | | | | | | |
| A25 | 0 | 1.5 | 0 | 1.875 | 0 | 5.625 | 0 | 3 | 202.0 ± 0.8 | 0.267 ± 0.012 | -20.3 ± 0.4 | 99.9 ± 0.1 |
| A26 | 0 | 1.5 | 0 | 1.875 | 0 | 5.625 | 0 | 3 | 199.3 ± 0.7 | 0.292 ± 0.038 | -19.6 ± 0.1 | 99.9 ± 0.1 |

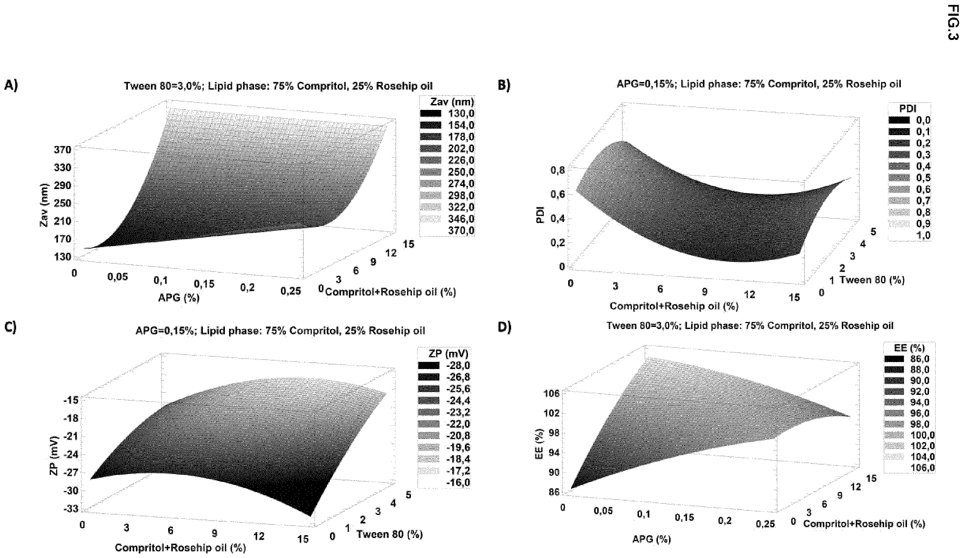

As it can be appreciated in Figure 3, the four variables studied had a significant effect on the formulation of the NLC. Average size and PDI of Loaded Lipid Nanoparticles are directly influenced by the concentration of glyceryl dibehenate + rosehip oil. Higher concentrations of glyceryl dibehenate + rosehip oil provide bigger Loaded Lipid Nanoparticles but lower PDI (Figure 3A, 3B). ZP is influenced by the amount of surfactant, a higher concentration of surfactant, less superficial charge is obtained (Figure 3C). EE is highly influenced by the proportion of glyceryl dibehenate added to the formulation. At higher glyceryl dibehenate concentrations, lower encapsulation of APG is obtained (Figure 3D). With these trends, the optimized formulation contains 0.1% of APG, 4.5% glyceryl dibehenate, 3.0% rosehip oil, and 3.5% of non-cationic surfactant.

To the optimized formulation, increasing amounts of the cationic surfactant were added (Table 2). The optimized formulation was chosen based on the physicochemical parameters, where values of ZP higher than 20 mV and PDI lower than 0.3 were selected. Because of that fact, the cationic optimized formulation was 0.05% of cationic surfactant.

Table 2. Effect of cationic surfactant on the physicochemical parameters.

| DDAB (%) | $Z_{av}$ ± SD (nm) | PDI ± SD | ZP ± SD (mV) |
|---|---|---|---|
| 0.025 | 301.8 ± 2.0 | 0.186 ± 0.003 | -0.8 ± 0.1 |
| **0.05** | **146.8 ± 0.8** | **0.253 ± 0.003** | **21.2 ± 0.4** |
| 0.06 | 144.3 ± 2.4 | 0.326 ± 0.036 | 27.3 ± 1.0 |
| 0.075 | 150.0 ± 3.8 | 0.363 ± 0.040 | 29.3 ± 0.5 |
| 0.1 | 148.2 ± 1.5 | 0.427 ± 0.018 | 35.6 ± 0.8 |
| 0.3 | 156.6 ± 1.9 | 0.407 ± 0.011 | 54.8 ± 1.6 |
| 0.5 | 147.7 ± 0.3 | 0.382 ± 0.009 | 54.4 ± 0.6 |

To this, 0.0001 % of HA was added and then, the optimized Loaded Lipid Nanoparticles were obtained (Table 3).

Table 3. Composition and physicochemical parameters of optimized formulation, Loaded Lipid
Nanoparticles

**Composition of Loaded Lipid Nanoparticles**

| | |
|---|---|
| APG | 0.1 % (w/v) |
| Glyceryl dibehenate | 4.5 % (w/v) |
| Rosehip oil | 3.0 % (w/v) |
| Polysorbate 80 | 3.5 % (w/v) |
| DDAB | 0.05 % (w/v) |
| HA | 0.0001 % (w/v) |

**Physycochemical parameters of Loaded Lipid Nanoparticles**

| $Z_{av} \pm SD$ (nm) | PI ± SD | ZP ± SD (mV) | EE ± SD (%) |
|---|---|---|---|
| 143.6 ± 1.3 | 0.254 ± 0.009 | 21.8 ± 0.6 | 99.9 ± 0.1 |

b) CHARACTERIZATION OF OPTIMIZED LOADED LIPID NANOPARTICLES

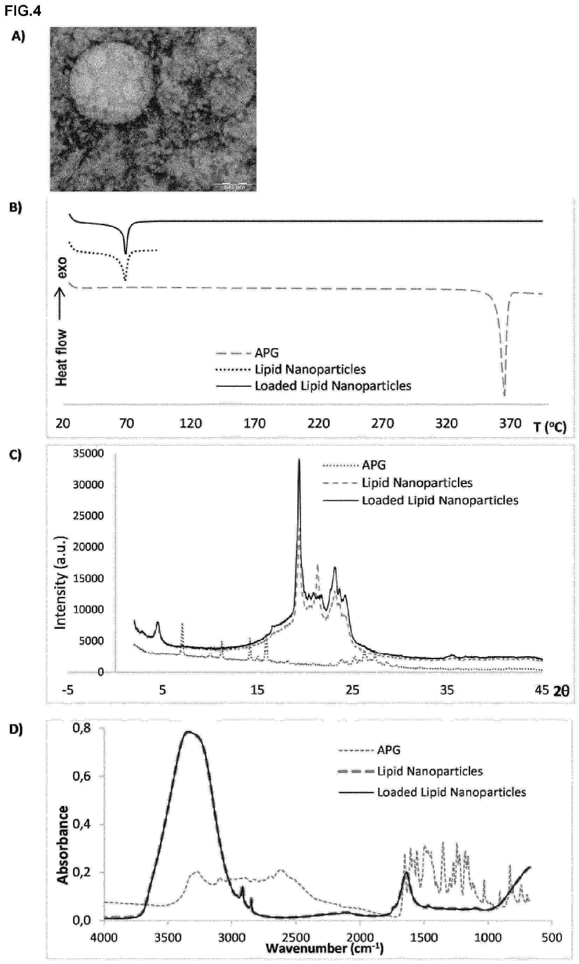

The morphology of Loaded Lipid Nanoparticles obtained by TEM shows almost spherical and soft shapes and the size below 200 nm is consistent with the results found by PCS. Particle aggregation phenomena is not observed (Figure 4A).

DSC was carried out in order to study the crystallinity and the melting point variations of the lipid mixtures and Loaded Lipid Nanoparticles. Thermogram (Figure 4B) shows endothermal peaks, of 70.37 °C for lipid mixture, 69.78 °C for lipid mixture-APG and 69.16 °C Loaded Lipid Nanoparticles. Melting point of Loaded Lipid Nanoparticles is slightly lower because of its small size and the addition of a surfactant (Polysorbate 80) in the formulation (28). The peaks move to slightly lower temperatures when APG is added and the enthalpy is similar between the lipid mixture and lipid mixture-APG being $\Delta$H Lipid mixture = 82.69 Jg$^{-1}$, $\Delta$H Lipid mixture-APG = 84.03 Jg$^{-1}$ and a smaller enthalpy for the nanoparticles, being $\Delta$H Loaded Lipid Nanoparticles = 54.11 Jg$^{-1}$. APG melting transition is characterized by an endothermal peak at 365.5°C ($\Delta$H = 198.5 Jg$^{-1}$) followed by decomposition [21].

XRD profiles in Figure 4C show the physical state of APG incorporated in NLC. Intense and sharp peaks for APG and for the solid mixture of lipids are shown, indicating that these components have a crystalline structure. The peaks found for APG are not detected in Loaded Lipid Nanoparticles profile, which could mean that the drug is present in a dissolved state in the NLC (molecular dispersion). The crystallinity of the structure of all the components was studied. The lipid mixture shows three peaks in 19.34 (2$\theta$), i.e. d=0,46 nm indicating the most stable form of triacylglycerols, the $\beta$ form, 21,28 (2$\theta$) i.e. d=0,42 nm and 23,43 (2$\theta$) i.e. d=0,38 nm, indicating the second stable form of triacylglycerols, the $\beta$' form. The Loaded Lipid Nanoparticles profile shows also the three peaks, which could indicate a good stability of the formulation [21-23].

FTIR analysis was used to study the interactions between the drug, the surfactant and lipid mixture (Figure 4D). The FTIR spectra of pure APG presented vibrational bands with characteristic peak at the wave number of 3278 cm$^{-1}$ for O-H group. However, C-H group presented multiple small peaks at 2800 cm$^{-1}$. The characteristic peaks at 1650 and 1605 cm$^{-1}$ were obtained for the C-O functional group [24]. There was no evidence of strong bonds between APG and lipid mixture and the surfactant. APG peaks were not found in the NLC. These results meaning that APG was encapsulated in the NLC.

Example 4: STABILITY STUDIES

Loaded Lipid Nanoparticles were stored at 4 and 25 °C during several months. The study was assessed analysing light backscattering (BS) profiles by a Turbiscan® Lab equipment. A glass measurement cell containing 20 ml of sample was used. Data were acquired every 30 days. The radiation source used was pulsed near-infrared light-

emitting diode LED ($\lambda$ = 880 nm), the signal was detected by a BS detector at an angle of 45° from the incident beam. At the same time interval, values of $Z_{av}$, PDI, ZP and EE were measured (20). The study is still running.

Stability studies were carried out by the backscattering (BS) profiles of each sample at different temperatures. BS profiles provides information of destabilization mechanisms in the media, such as sedimentation, agglomeration, or aggregation [25]. In this way, BS profiles of Loaded Lipid Nanoparticles were studied at 4 °C and 25 °C. The Loaded Lipid Nanoparticles formulation is stable at 4 °C for a period of 19 months, while at 25 °C the stability endures 2 months. The physicochemical parameters kept constant at 4 °C for all the study. The best storage temperature is at 4 °C.

Example 5: BIOPHARMACEUTICAL BEHAVIOUR

The in vitro APG release test for Loaded Lipid Nanoparticles was performed using Franz-type diffusion cells (Crown Glass, NY, USA) with a diffusion area of 0.20 cm$^2$ and dialysis membranes of cellulose (MWCO 12 kDa). A solution of PBS with 5% polysorbate 80 and 20% ethanol under continuous stirring was used as a receptor medium assuring sink conditions (ability of the medium to dissolve the expected amount of drug) [26]. The formulations were compared with free-APG solution. The assay was carried out at 32 $\pm$ 0.5 °C along 48h. 300 $\mu$l of each formulation were added to the donor compartment by direct contact with the membrane. At a certain timepoints, 150 $\mu$l of sample were collected with a syringe and the volume withdrawn was replaced with receptor solution. Drug content of the samples was analysed with HPLC. The test was performed by triplicate, and the cumulative amount of APG was calculated [16].

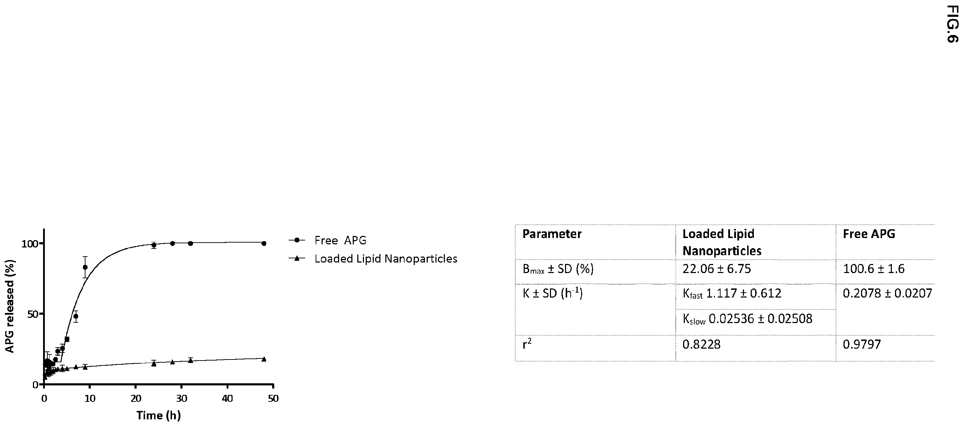

The in vitro release profile of APG from the NLC demonstrates that the formulation has a kinetic profile that is characteristic of prolonged drug release formulations. The release best fit for NLC was two phase decay. Figure 6 shows a faster release of APG from the NLC during the first 8h, after that the speed was decreased. The free APG had a faster release, achieving a 100% at 24h while the Loaded Lipid Nanoparticles released less than 20%. It has been reported that modifying the surface of nanocarriers with hyaluronic acid can restrict water diffusion into the carrier matrix which subsequently slows down the drug release process [27]. These results indicated the formulation had a prolonged release of APG.

Example 6: OCULAR TORELANCE

6.1 In vitro study: HET-CAM test and TBS

In vitro ocular tolerance was assessed using the HET-CAM test to ensure that the formulations of Loaded Lipid Nanoparticles were non-irritating when administered as eye-drops. Irritation, coagulation, and haemorrhage phenomena were measured by applying 300 $\mu$L of the formulation studied on chorioallantoic membrane of a fertilized chicken egg and monitoring it during the first 5 min after the application. This assay was conducted according to the guidelines of ICCVAM (The Interagency Coordinating Committee on the Validation of Alternative Methods). The development of the test was carried out using 3 eggs for each group (free APG, Loaded Lipid Nanoparticles, positive control (NaOH 0.1 M) and negative control (0.9 % NaCl)). The ocular irritation index (OII) was calculated by the sum of the scores of each injury according to the following expression (Eq 3) [18]:

$$OII = \frac{(301-H)\cdot 5}{300} + \frac{(301-V)\cdot 7}{300} + \frac{(301-C)\cdot 9}{300} \qquad \text{Eq. 3}$$

where H, V and C are times (s) until the start of haemorrhage (H), vasoconstriction (V) and coagulation (C), respectively. The formulations were classified according to the following: OII $\leq$ 0.9 non-irritating; 0.9 < OII $\leq$ 4.9 weakly irritating; 4.9 < OII $\leq$ 8.9 moderately irritating; 8.9 < OII $\leq$ 21 irritating.

Furthermore, at the end of the HET-CAM experiment, in order to quantify the damage of the membrane, trypan blue staining (TBS) was applied. The CAM was treated with 1000 $\mu$L of 0.1% trypan blue solution for 1 min. Excess dye was rinsed off with distilled water. The dyed CAM was excised and extracted with 5 mL formamide, and the absorbance of the extract was measured spectrophotometrically at 595 nm. The absorbed trypan blue was determined from a calibration curve of trypan blue in formamide [28].

HET-CAM test was applied showing that the positive controls (NaOH 1 M) resulted in severe haemorrhage, which increased over five minutes grading this solution as a severe irritant. On the other hand, application of the formulations to the chorioallantoic membrane did not cause irritation and therefore, the formulations were classified as non-irritant. Moreover, TBS quantitative results supported the results of the HET-CAM test, where Loaded Lipid Nanoparticles were non-irritant while free APG resulted irritant.

6.2 In vivo study: Draize test

All of the procedures were approved by the Ethical Committee for Animal Experimentation of the UB and current legislation (Decree 214/97, Gencat). The formulations were evaluated using primary eye irritation test of Draize to ensure the results obtained from the HEM-CAM test. For this experiment, New Zealand male albino rabbits (2.0-2.5 kg, San Bernardo farm, Navarra, Spain) were used. 50 $\mu$L of each sample were instilled in the ocular conjunctival sac (n = 3/group) and a mild massage was applied to guarantee the passage of the sample through the eyeball. The possible appearance of irritation signs (corneal opacity and area of corneal involvement, conjunctival hyperemia, chemosis, ocular discharges, and iris abnormalities) was observed at the time of instillation and after 1 h from its application and if necessary, at predefined intervals: 24 h, 48 h, 72 h, 7 days, and 21 days after administration. The opposite untreated eye was used as a negative control. Draize test score was determined directly by observing the anterior segment of the eye and changes in the structures of the cornea (turbidity or opacity), iris and conjunctiva (congestion, chemosis, swelling and secretion) [2].

The tests were carried out with free APG, Lipid Nanoparticles, Loaded Lipid Nanoparticles. In this sense, none of the developed NLC were irritant in vivo or in vitro, while the free APG resulted irritant in vitro and non-irritant in vivo but caused an initial discomfort. These results confirmed the non-irritant potential of APG loaded lipid nanoparticles, meanwhile the free APG can induce some discomfort.

Example 7: IN VIVO EFFICACY STUDIES

In order to evaluate the potential to treat dry eye of Loaded Lipid Nanoparticles, Schirmer test, fluorescein and rose Bengal assessments were performed against Lipid Nanoparticles, free APG and 0.15% hyaluronic acid (commercial solution Hyabak®).

7.1 Induction and treatment of dry eye

Twelve male New Zealand white rabbits (purchased from Livestock Research Institute, Council of Agriculture, Executive Yuan, Taiwan) weighing between 2.0 and 2.5 kg were used for the study. The rabbits were randomly divided into 4 groups: Lipid Nanoparticles, Loaded Lipid Nanoparticles, free APG solution and 0.15% hyaluronic acid (commercial solution (Hyabak®). All rabbits were housed at a room temperature of 23° $\pm$ 2 °C with relative humidity 75% $\pm$ 10% and alternating 12-hour light-dark cycles (8 a.m. to 8 p.m.). Both eyes of each rabbit were treated twice-daily by a topical administration of 0.1% benzalkonium chloride (BAC) drops for 2 weeks. On day 14, DES was confirmed by Schirmer test, fluorescein and rose Bengal staining. The treatment began after the confirmation of DES, where on eye of each rabbit was chosen randomly for twice-daily topical administration of Lipid Nanoparticles, Loaded Lipid Nanoparticles, free APG solution and a commercial solution. After one week of treatment, Schirmer test, fluorescein and rose Bengal staining were performed [2,29].

7.1.1. Measurement of aqueous tear production

Tear production was measured using Schirmer test strips. After the topical application of anaesthetic drops (1 mg/ml tetracaine hydrochloride/4 mg/ml oxybuprocaine hydrochloride), the lower eyelid was pulled down, and a Schirmer paper strip was placed on the palpebral conjunctiva near the junction of the middle and outer thirds of the lower eyelid. After 5 min, the wetted length (mm) of the paper strips was recorded [2,29].

A severe decrease in the aqueous tear secretion was achieved after the application of benzalkonium chloride for 2 weeks. Figure 7 shows the differences between each group of treatment. Lipid Nanoparticles and Loaded Lipid Nanoparticles were able to increase the tear flow in the animals, with statistically significant differences against dry eye group, $p < 0.01$ and $p < 0.0001$ respectively. Otherwise, free APG and the commercial solution did not improve the tear flow. Loaded Lipid Nanoparticles showed statically significant differences between all the groups: $p < 0.01$ against Lipid Nanoparticles, and $p < 0.0001$ against free APG and commercial solution. Lipid Nanoparticles showed statistically significant differences between free APG group ($p < 0.01$). Loaded Lipid Nanoparticles were the treatment that attained the best score in the Schirmer test, followed by the Lipid Nanoparticles, meaning that both were able to restore the tear secretion of the animals. These results reveal the potential of the composition of the nanoformulations because of the restoring of the tear flow in the animals. Loaded Lipid Nanoparticles presented a better score due to the encapsulation of APG, which it has anti-inflammatory properties, leading to a better improvement of one of the symptoms of DED.

7.1.2. Fluorescein staining on the ocular surface

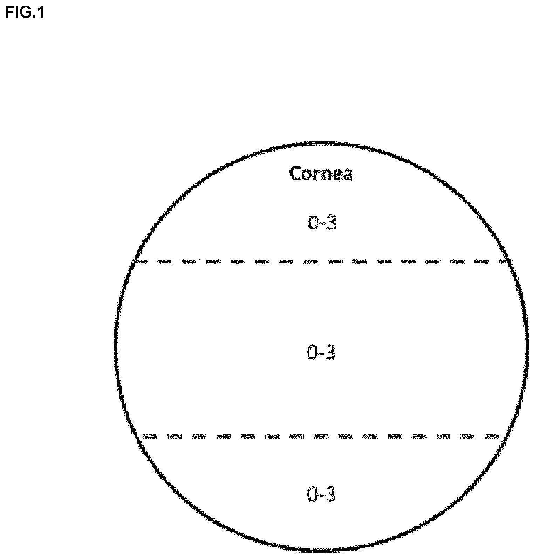

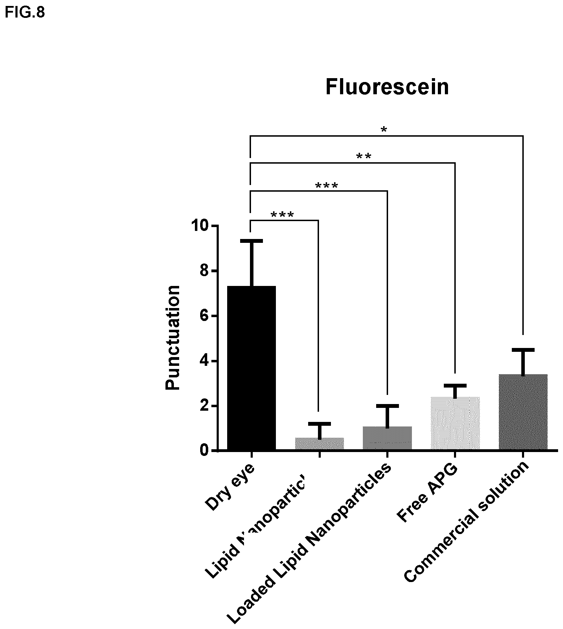

Fluorescein staining is an effective method for ocular surface evaluation. Fluorescein staining is the result of uptake caused by the disruption of corneal epithelial cell-cell junctions or damaged corneal epithelial cells [29]. Corneal fluorescein staining was performed after 2 µL of 1 % fluorescein sodium were dropped into the conjunctival sac for 2 min. The ocular surface was examined under a slit lamp microscope with a cobalt blue filter. The images were collected by a digital camera (Figure 1) and punctuated according to the stained score (9: maximum score; 0: minimum)[30]. Figure 8 shows the differences between each group of treatment. There were statistically significant differences between all the groups against dry eye control. However, the two treatments that had a better improvement of the ocular corneal surface were Lipid Nanoparticles and Loaded Lipid Nanoparticles (p < 0.001). Moreover, free APG solution and the commercial solution presented worse punctuation in the staining, meaning that both did not have the ability to restore the corneal surface as Lipid Nanoparticles and Loaded Lipid Nanoparticles.

7.1.3. Bengal rose staining on the ocular surface

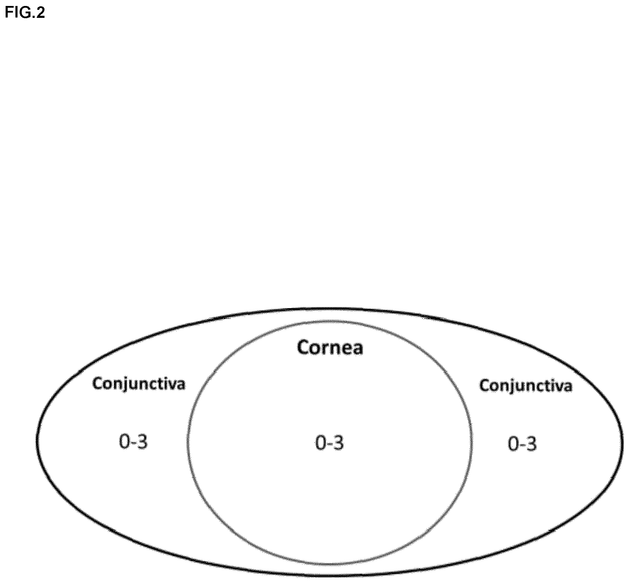

Bengal rose is an effective method to evaluate the tear film integrity. Bengal rose has been demonstrated to stain corneal and conjunctival epithelial cells that are not adequately protected by the preocular tear film. It can stain live and dead cells if they are not protected by an intact mucin layer [29]. Ocular fluorescein staining was performed after 2 µL of 0.1% Bengal rose were dropped into the conjunctival sac for 2 min. The ocular surface was examined under a slit lamp microscope with a white light. The images were collected by a digital camera. Using the Van Bijsterveld grading system, the scores were graded after 15 seconds (Figure 2) (9: maximum score; 0: minimum) [29].

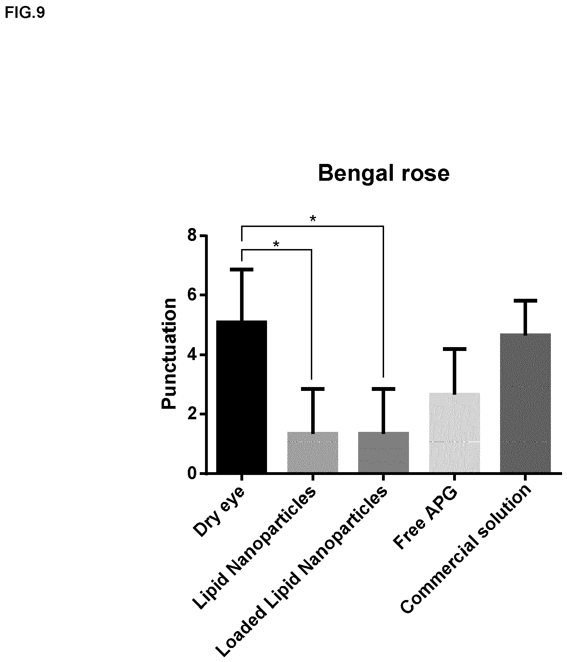

There were statistically significant differences between dry eye and Lipid Nanoparticles and Loaded Lipid Nanoparticles intensity (Figure 9). The results showed that only NLC were able to restore tear film in the animals. The worst punctuation for the rose Bengal staining was the commercial solution. Thus, indicating that Loaded Lipid Nanoparticles were able to restore the tear film.

These results showed the ability of the lipid nanoparticles to revert the symptoms of the DED. Lipid Nanoparticles and Loaded Lipid Nanoparticles were able to improve all the studied parameters of DED. These results could be due to the novel composition of the lipid nanoparticles. The addition of the rosehip oil provides anti-inflammatory and antioxidant properties to the Lipid Nanoparticles [31,32]. Because of this fact, Lipid Nanoparticles improves the score of both different staining of the ocular surface, showing interesting properties against DED. The addition of the APG had improved the tear secretion on the Schirmer test, which could mean that NLC protects APG to be released and enhance the anti-inflammatory properties of the formulation, improving the DED symptomatology.

7.1.4 Anti-inflammatory efficacy

In vivo anti-inflammatory efficacy was assayed to explore the capacity of the NLC to prevent and treat ocular inflammation through two different tests. The assays were carried out throughout the evaluation test for the inflammation prevention ability and the anti-inflammatory efficacy using New Zealand male albino rabbits (n = 3/group), described previously. The activity of Loaded Lipid Nanoparticles in comparison with free APG and NaCl 0.9% (control group) was measured. The inflammation prevention study consisted of the ocular application of 50 µL of each formulation.

After 30 min of exposure, an inflammatory stimulus, 50 µL of 0.5% sodium arachidonate (SA) dissolved in PBS, was instilled in the right eye and the left eye was used as a control. In the anti-inflammatory treatment study, the inflammatory stimulus was applied 30 min before than the application of each formulation tested. The evaluation of prevention and treatment of each formulation were carried out from the first application up to 210 min, according to the Draize modified test scoring system [2,18].

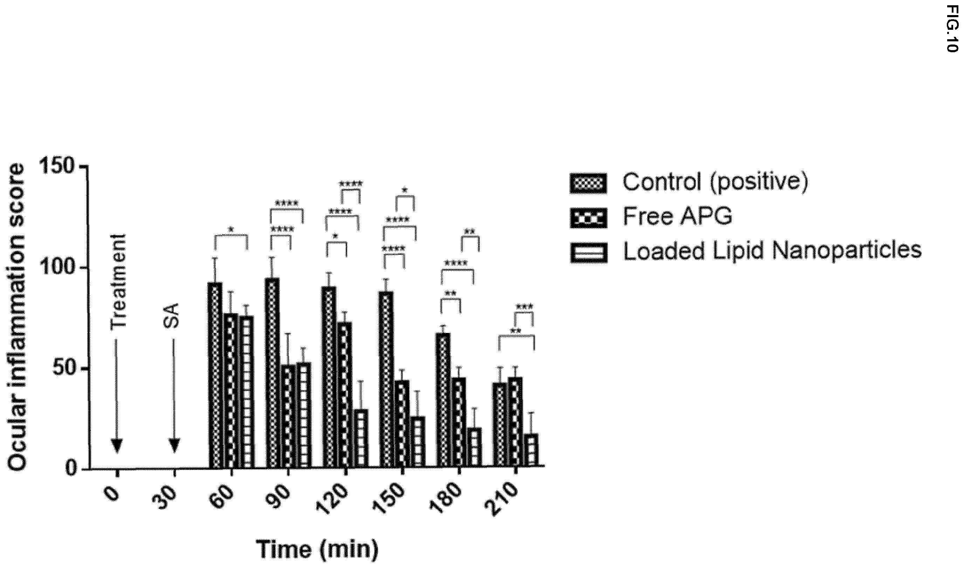

In vivo inflammatory prevention test showed significant differences between the degree of inflammation of APG formulations or physiological serum during all the timepoints tested (Figure 10). Nevertheless, eyes treated with Loaded Lipid Nanoparticles presented a faster swelling reduction rather than free APG, mainly owing to tear clearance in case of free APG and the improved ocular surface adherence of lipid nanoparticles, thus presenting longer residence time in the cornea. Loaded Lipid Nanoparticles exhibited significant differences regarding positive control over the time. Thus, Loaded Lipid Nanoparticles exhibited a preventive effect of inflammation caused by the sustained release of APG to the corneal cells.

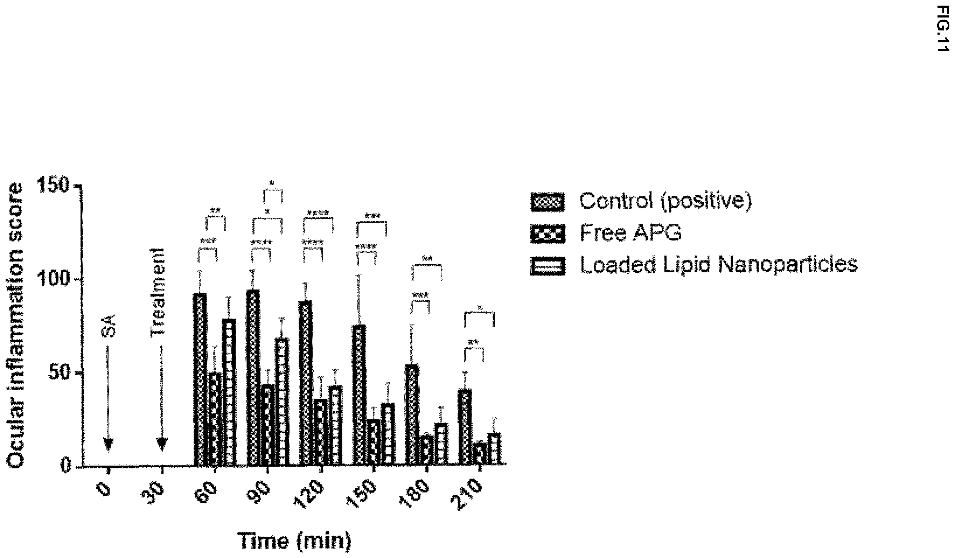

In addition, the in vivo inflammation treatment was assessed. Loaded Lipid Nanoparticles and free APG were applied after 30 min of SA exposure, and the degree of inflammation was quantified. Figure 11 revealed that the degree of inflammation was significantly reduced after the first 90 minutes post-administration of Loaded Lipid Nanoparticles. Free APG were able to treat faster inflammation due to the NLC had a controlled release. After 90 min of contact, both APG formulations were effective in treating inflammation symptoms. Hence, it can be concluded that the controlled release system based on Loaded Lipid Nanoparticles has ocular anti-inflammatory activity, both for prevention level and inflammation treatment.

## 7.1.5 STATISTICAL ANALYSIS

Two-way ANOVA followed by Tukey post hoc test was performed for multi-group comparison. Student's t-test was used for two-group comparisons. All the data are presented as the mean $\pm$ S.D. Statistical significance was set at $p < 0.05$ by using GraphPad Prism 6.0.1.

## REFERENCES

[1] Hantera MM. Trends in Dry Eye Disease Management Worldwide. Clin Ophthalmol 2021;15:173.

[2] López-Machado A, Díaz-Garrido N, Cano A, Espina M, Badia J, Baldomà L, et al. Development of Lactoferrin-Loaded Liposomes for the Management of Dry Eye Disease and Ocular Inflammation. Pharmaceutics 2021;13 (10):1698.

[3] Huang L, Gao H, Wang Z, Zhong Y, Hao L, Du Z. Combination Nanotherapeutics for Dry Eye Disease Treatment in a Rabbit Model. Int J Nanomedicine 2021;16:3631.

[4] Salehi B, Venditti A, Sharifi-Rad M, Kregiel D, Sharifi-Rad J, Durazzo A, et al. The Therapeutic Potential of Apigenin. Int J Mol Sci 2019;20 (6):1305.

[5] Ali F, Rahul, Naz F, Jyoti S, Siddique YH. Health functionality of apigenin: A review. Int J Food Prop 2016;20 (6):1197-238.

[6] Clinical Trial to Evaluate the Reduction of Cardiovascular Risk - ClinicalTrials.gov n.d. https://clinicaltrials.gov/ct2/show/NCT04114916?term=apigenin&draw=4&rank=3 (accessed November 19, 2021).

[7] Zick SM, Wright BD, Sen A, Arnedt JT. Preliminary examination of the efficacy and safety of a standardized chamomile extract for chronic primary insomnia: a randomized placebo-controlled pilot study. BMC Complement Altern Med 2011;11:78.

[8] Apigenbell 60 cápsulas Jellybell | Naturitas n.d. https://www.naturitas.es/p/suplementos/fitoterapia/compuestos-herbarios/apigenbell-60-capsulas-jellybell?gclid=CjwKCAiAs92MBhAXEiwAXTi25z_Q5qx2aeNYvzbrUCyGRG6PfdA91wTKXvt WHr461rXXYtFP_cDcZxoCkrAQAvD_BwE (accessed November 19, 2021).

[9] Apigenina 90 cápsulas de 50mg Swanson | Naturitas n.d. https://www.naturitas.es/p/suplementos/suplementos-compuestos/apigenina-90-capsulas-de-50mg-swanson (accessed November 19, 2021).

[10] Optrex Colirio para el Picor de Ojos n.d. https://www.optrex.es/gama-optrex/colirio/optrex-colirio-calmante-para-el-picor-de-ojos/optrex-colirio-calmante-para-el-picor-de-ojos/ (accessed November 19, 2021).

[11] Optiben - Irritación ocular n.d. https://optiben.cmfa.com/Producto/7/alivio-natural-de-la-irritacin-ocular (accessed November 19, 2021).

[12] Wang M, Firrman J, Liu LS, Yam K. A review on flavonoid apigenin: Dietary intake, ADME, antimicrobial effects, and interactions with human gut microbiota. Biomed Res Int 2019;7010467.

[13] Battaglia L, Serpe L, Foglietta F, Muntoni E, Gallarate M, Del Pozo Rodriguez A, et al. Application of lipid nanoparticles to ocular drug delivery. Expert Opin Drug Deliv 2016;13 (12):1743-57.

[14] Fangueiro JF, Calpena AC, Clares B, Andreani T, Egea MA, Veiga FJ, et al. Biopharmaceutical evaluation of epigallocatechin gallate-loaded cationic lipid nanoparticles (EGCG-LNs): In vivo, in vitro and ex vivo studies. Int J Pharm 2016;502 (1-2):161-9.

[15] Agarwal P, Craig JP, Rupenthal ID. Formulation Considerations for the Management of Dry Eye Disease. Pharmaceutics 2021;13 (2):207.

[16] Carvajal-Vidal P, Fábrega MJ, Espina M, Calpena AC, Garcia ML. Development of Halobetasol-loaded nanostructured lipid carrier for dermal administration: Optimization, physicochemical and biopharmaceutical behavior, and therapeutic efficacy. Nanomedicine Nanotechnology, Biol Med 2019;20:102026.

[17] Sánchez-López E, Ettcheto M, Egea MA, Espina M, Cano A, Calpena AC, et al. Memantine loaded PLGA PEGylated nanoparticles for Alzheimer's disease: In vitro and in vivo characterization. J Nanobiotechnology 2018;16 (1):1-16.

[18] Sánchez-López E, Esteruelas G, Ortiz A, Espina M, Prat J, Muñoz M, et al. Dexibuprofen Biodegradable Nanoparticles: One Step Closer towards a Better Ocular Interaction Study. Nanomaterials 2020;10 (4).

[19] Romanová D, Grancai D, Józová B, Bożek P, Vachálková A. Determination of apigenin in rat plasma by high-performance liquid chromatography. J Chromatogr A 2000;870 (1-2):463-7.

[20] Zeb A, Qureshi OS, Kim HS, Kim MS, Kang JH, Park JS, et al. High payload itraconazole-incorporated lipid nanoparticles with modulated release property for oral and parenteral administration. J Pharm Pharmacol 2017;69 (8):955-66.

[21] Souto EB, Mehnert W, Müller RH. Polymorphic behaviour of Compritol888 ATO as bulk lipid and as SLN and NLC. J Microencapsul 2006;23 (4):417-33.

[22] Freitas C, Müller RH. Correlation between long-term stability of solid lipid nanoparticles (SLN) and crystallinity of the lipid phase. Eur J Pharm Biopharm 1999;47 (2):125-32.

[23] Zimmermann E, Souto EB, Müller RH. Physicochemical investigations on the structure of drug-free and drug-loaded solid lipid nanoparticles (SLN) by means of DSC and 1H NMR. Pharmazie 2005;60 (7):508-13.

[24] Alshehri SM, Shakeel F, Ibrahim MA, Elzayat EM, Altamimi M, Mohsin K, et al. Dissolution and bioavailability improvement of bioactive apigenin using solid dispersions prepared by different techniques. Saudi Pharm J 2019;27 (2):264-73.

[25] Stability of Dispersions | 3P Instruments n.d. https://www.3p-instruments.com/measurement-methods/stability-turbiscan/ (accessed November 22, 2021).

[26] Liu P, De Wulf O, Laru J, Heikkilä T, Van Veen B, Kiesvaara J, et al. Dissolution Studies of Poorly Soluble Drug Nanosuspensions in Non-sink Conditions. AAPS PharmSciTech 2013;14 (2):748.

[27] Huang D, Chen YS, Rupenthal ID. Hyaluronic Acid Coated Albumin Nanoparticles for Targeted Peptide Delivery to the Retina. Mol Pharm 2017;14 (2):533-45.

[28] Lagarto A, Vega R, Guerra I, González R. In vitro quantitative determination of ophthalmic irritancy by the chorioallantoic membrane test with trypan blue staining as alternative to eye irritation test. Toxicol In Vitro 2006;20 (5):699-702.

[29] Xiong C, Chen D, Liu J, Liu B, Li N, Zhou Y, et al. A rabbit dry eye model induced by topical medication of a preservative benzalkonium chloride. Invest Ophthalmol Vis Sci 2008;49 (5):1850-6.

[30] Holzchuh R, Villa Albers MB, Osaki TH, Igami TZ, Santo RM, Kara-Jose N, et al. Two-year outcome of partial lacrimal punctal occlusion in the management of dry eye related to Sjögren syndrome. Curr Eye Res 2011;36 (6):507-12.

[31] Lin TK, Zhong L, Santiago JL. Anti-Inflammatory and Skin Barrier Repair Effects of Topical Application of Some Plant Oils. Int J Mol Sci 2017;19 (1):70.

[32] Kiralan M, Yildirim G. Rosehip (Rosa canina L.) Oil. Fruit Oils Chem Funct 2019:803-14.

**Claims**

1. A composition comprising lipid nanoparticles, said lipid nanoparticles comprising

   a. at least one liquid lipid with anti-inflammatory properties;
   b. at least one solid lipid;
   c. at least one cationic surfactant;
   d. optionally, at least one non-cationic surfactant;
   e. optionally, at least one active ingredient.

2. The composition of claim 1, wherein the said liquid lipid with anti-inflammatory properties is selected from the group consisting of rosehip oil, tea tree oil, lavender oil, linoleic acid, stearic acid, palmitic acid, castor oil, safflower oil, melon seed oil, salicornia oil, evening primrose oil, poppyseed oil, grape seed oil, prickly pear oil, artichoke oil, hemp oil, wheat germ oil, cottonseed oil, corn oil, walnut oil, soybean oil, sesame oil, rice bran oil, argan oil, pistachio oil, peach oil, almond oil, canola oil, avocado oil, flaxseed oil, sunflower oil, peanut oil, palm oil, olive oil, macadamia oil, coconut oil, rosemary oil, lavender oil, origanum vulgare oil, thyme oil, mint oil, eucalyptus oil, ginger oil, cuminum cyminum l. oil, turmeric oil, clove oil, oleic acid, oregano oil, rose oil, fennel oil, bergamot oil, chamomile oil, helichrysum oil, patchouli oil, frankincense oil, copaiba oil, peppermint oil, black pepper oil, sweet marjoram oil, basil oil, clove oil, clary sage oil, lemongrass oil, geranium oil, wintergreen oil, cannabis oil, cannabidiol, spruce oil, niaouli oil, cardamom oil, pine tree oil, fir tree oil, juniper tree oil, verbena oil, marjoram oil, katafray oil, bitter orange oil, hypericum oil, arnica oil, coriander oil, mustard oil, perilla seed oil, centella asiatica oil, calendula oil, laurel oil, camphor oil, cinnamon oil, oatmeal oil, docosahexaenoic acid, eicosapentaenoic acid, dandelion oil, krill oil, electrophorus electricus oil, potamotrygon motoro oil, boa constrictor oil, chelonoidis denticulate oil, melanosuchus niger oil, inia geoffrensis oil, horse oil, anchovy oil, prunus seed oil, tropidurus hispidus oil, emu oil, maqian fruits essential oil, fructus alpinia oil, cinnamomum cassia essential oil, angelica sinensis oil, gynura procumbens oil, spirulina oil, citrus limetta oil, citrus aurantium oil, atractylodes macrocephala oil, artemisia argyi oil, gynura procumbens oil, acorus gramineusand oil, algal oil, fish oil, zanthoxylum coreanum nakai oil, cod liver oil, perna canaliculus oil, chia seed oil, or combinations thereof.

3. The composition of claim 1, wherein the liquid lipid with anti-inflammatory properties is rosehip oil.

4. The composition of any one of the preceding claims, wherein the solid lipid is selected from monoglycerides, diglycerides, triglycerides, cholesterols, steroids, fatty alcohols, glycerol esters glyceryl tridecanoate, glycerol trilaurate, glyceryl trimyristate, glyceryl tripalmitate, glyceryl tristearate, Hydrogenated coco-glycerides, Hard fat types, mixtures of triglycerides and/or diglycerides and/or monoglycerides and/or glycerol, acyl glycerols, glyceryl monostearate, glyceryl distearate, glyceryl monooleate, glyceryl dibehenate, glyceryl palmitostearate, waxes, cetyl palmitate, fatty acids, stearic acid, palmitic acid, decanoic acid, behenic acid, glycerol stearate citrate, polyethylene glycol monostearate, cyclic complexes, cyclodextrin para-acyl-calix-arenes, or mixtures thereof.

5. The composition of any one of the preceding claims, wherein the cationic surfactant is selected from the group consisting of dimethyldioctadecylammonium bromide (DDAB), dioleoyl phosphatidylethanolamine, 1,2-distearyloxy-N,N-dimethyl-3-aminopropane, 1,2 dioleyl-oxy-N,N-dimethyl-3-aminopropane,1,2-dilinoleyloxy-N,N-dimethyl-3-aminopropane, 1,2 - dilinolenyloxy-N,N-dimethyl-3- aminopropane, cetyltri-methylammonium bromide, 3β-[N(N',N'-dimethylaminoethane)-carbamoyl]cholesterol, 1,2-dioleoyl-3-trimethylammo-nium-propane, 1,2-dimyristoyl-3-tri-methylammonium-propane, 1,2-stearoyl-3-tri-methylammonium-propane, N-(4-carboxybenzyl)-N,N-dimethyl-2,3-bis(oleoyloxy) propan-1-aminium, 1-Palmitoyl-2-oleoyl-sn-glycero-3-phosphoethanol-amine, N,N-di-(β-stearoylethyl)-N,N-dimethyl-ammonium chloride, benzalkonium chloride, cetylpyridinium chloride, cetrimide, N-[1-(2,3-dioleoyloxy)propyl]-N,N,N-trimethylammonium chloride, 1,2-dilineoyl-3-dimethylammonium-propane, 1,2-dilinoleyloxy-3-N,N-dimethylaminopropane, 1,2-dilinoleyloxy- keto-N,N-dimethyl-3-aminopropane, 1,2-dilinoleyl-4-(2- dimethylaminoethyl)-[1,3]-dioxolane(3-o-[2"-(meth oxypolyethyleneglycol 2000) succinoyl]-1,2-dimyristoyl-sn-glycol, R-3-[(ω-methoxy-poly(ethyleneglycol)2000) carbamoyl]-1,2-dimyristyloxlpropyl-3-amine, cetyltrimethylammonium bromide, octadecylamine, 1-oleoyl-rac-glycerol, octadecyl quaternized carboxymethyl chitosan, hexadecyl trimethyl ammonium bromide.

6. The composition of any one of the preceding claims, wherein the cationic surfactant is dimethyl-dioctadecyl-ammonium bromide (DDAB).

7. The composition of any one of the preceding claims, wherein the non-cationic surfactant is selected from the group consisting of polysorbate 80, soya lecithin, sodium dodecyl sulphate, polysorbate 20, polysorbate 40, polysorbate 60, PEG-30 glyceryl stearate, cholic acid, phosphatidyl choline, phospholipids with phosphatidycholine, egg lecithin, poloxamer 188, poloxamer 407, poloxamer 184, poloxamer 338, poloxamine 908, tyloxopol, taurocholate sodium salt, taurodeoxycholicacid sodium salt, sodium glycocholate, sodium oleate, cholesteryl hemisuccinate, butanol, sodium cholate, nonionic polyoxyethylene, non-ionic am-phiphilic surfactant with an alkyl moiety and an ethylene oxide chain, palmitic acid, stearic acid, mixtures of palmitic and stearic acid, lecithin, polyglycerol 6-distearate, caprylyl/capryl glucoside, coco-glucoside, sucrose palmitate, sucrose stearate, sucrose distearate, tyloxapol, lecithin, cetylpyridinium chloride, sorbitan laurate, polyethylene glycol ether of cetyl or stearyl alcohol, castor oil polyoxyethylene ether, macrogolglycerol ricinoleate, dioctyl sodium sulfosuccinate, monooctylphosphoric acid sodium, hexadecyl trimethyl ammonium bromide, polyvinyl alcohol, polyoxyethylene (40) stearate, polyethylene glycol-polypropylene glycol-polyethylene glycol triblock copolymer, olyoxyethylene nonylphenyl ether, hexadecyltrimethylammonium bromide, sodium dodecyl sulfate, sodium cholate, stearate sodium hydrolysed polyvinyl alcohol 9000-10000 MW, dioctyl sulfosuccinate, taurocholate, 4-dodecylbenzenesulfonic acid, long chain carboxylic acid, alkyldiphenyloxide disulfonate, or mixtures thereof, preferably polysorbate 80.

8. The composition of any one of the preceding claims, wherein the at least one active ingredient is apigenin.

9. The composition of any one of the preceding claims, wherein the at least one active ingredient is encapsulated in the lipid nanoparticles.

10. The composition of any one of the preceding claims, wherein said lipid nanoparticles further comprise a coating, preferably wherein said coating comprises hyaluronic acid.

11. The composition of any one of the preceding claims, wherein said lipid nanoparticles comprise

    a. 0.01 - 30 % (w/v) liquid lipid with inflammatory properties, preferably rosehip oil
    b. 1 - 50 % (w/v) solid lipid, preferably glyceryl dibehenate;
    c. 0.001 - 10 % (w/v) cationic surfactant, preferably DDAB;
    d. optionally 0.00 - 10 % (w/v) non-cationic surfactant, preferably polysorbate 80;
    e. optionally 0.00 - 10 % (w/v) active ingredient, preferably apigenin.

**12.** The composition of any one of the preceding claims, wherein said lipid nanoparticles comprise

    a. 2.5 - 12.5% (w/v) liquid lipid with inflammatory properties, preferably rosehip oil
    b. 1 - 30 % (w/v) solid lipid, preferably glyceryl dibehenate;
    c. 0.025 - 0.5 % (w/v) cationic surfactant, preferably DDAB;
    d. 1.0 - 5.0% (w/v) non-cationic surfactant, preferably polysorbate 80;
    e. optionally 0.1 - 2.5% (w/v) active ingredient, preferably apigenin.

**13.** The composition of any one of the preceding claims, wherein said lipid nanoparticles comprise

    a. 3.0% (w/v) rosehip oil,
    b. 4.5 % (w/v) glyceryl dibehenate,
    c. 0.05% (w/v) DDAB,
    d. 3.5% (w/v) polysorbate 80,
    e. optionally 0.1% (w/v) apigenin.

**14.** The composition of any one of the preceding claims, wherein the lipid nanoparticles further comprise from 0.00001 % - 0.001 % (w/v), preferably 0.0001 % (w/v) hyaluronic acid, more preferably wherein said hyaluronic acid coats the lipid nanoparticles.

**15.** A composition comprising lipid nanoparticles according to any one of the preceding claims for use in the treatment, amelioration or prevention of ocular diseases, such as ocular inflammation, glaucoma, ocular tumors, bacterial and viral infections of the eye, age-related macular degeneration, cataracts, diabetic retinopathy, or Dry Eye Disease (DED).

**FIG.1**

FIG.2

FIG.3

FIG.4

A)

B)

C)

D)

FIG.5

A)

B)

FIG.6

| Parameter | Loaded Lipid Nanoparticles | Free APG |
|---|---|---|
| $B_{max} \pm SD$ (%) | 22.06 ± 6.75 | 100.6 ± 1.6 |
| $K \pm SD$ (h$^{-1}$) | $K_{fast}$ 1.117 ± 0.612 | 0.2078 ± 0.0207 |
| | $K_{slow}$ 0.02536 ± 0.02508 | |
| $r^2$ | 0.8228 | 0.9797 |

FIG.7

Schirmer test

FIG.8

**Fluorescein**

FIG.9

Bengal rose

FIG.10

FIG.11

Europäisches
Patentamt

European
Patent Office

Office européen
des brevets

**EUROPEAN SEARCH REPORT**

Application Number

EP 23 38 2105

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALMEIDA HUGO ET AL: "Preparation, characterization and biocompatibility studies of thermoresponsive eyedrops based on the combination of nanostructured lipid carriers (NLC) and the polymer Pluronic F-127 for controlled delivery of ibuprofen", PHARMACEUTICAL DEVELOPMENT AND TECHNOLOGY, vol. 22, no. 3, 3 April 2017 (2017-04-03), pages 336-349, XP093066262, US ISSN: 1083-7450, DOI: 10.3109/10837450.2015.1125922 Retrieved from the Internet: URL:http://dx.doi.org/10.3109/10837450.2015.1125922> | 1,2,4,5, 7,9,11, 12,15 | INV. A61K9/51 A61K9/00 A61K36/738 A61K31/352 A61P27/00 A61P27/06 A61P27/12 A61P31/04 A61P31/12 A61P35/00 |
| Y | * abstract * * page 338, right-hand column, paragraph – page 339, left-hand column, paragraph 1; table 1 * * page 336, right-hand column, paragraph 2 * * page 338, left-hand column, paragraph Materials * ----- | 8,10,13, 14 | |
| X | WO 2022/235238 A2 (DR SEYDA ATABAY SAGLIK VE KOZMETIK UERUENLERI A S [TR]) 10 November 2022 (2022-11-10) | 1-5,7, 9-12 | TECHNICAL FIELDS SEARCHED (IPC) A61K |
| Y | * page 10, line 7 – page 13, line 4; claims 1-18 * ----- | 10,13,14 | |
| X | WO 2011/116963 A2 (LIPOTEC SA [ES]; VILADOT PETIT JOSEP LLUIS [ES] ET AL.) 29 September 2011 (2011-09-29) * examples 3-4-5,7 * ----- -/-- | 1,2,4, 7-12,14 | |

The present search report has been drawn up for all claims

| Place of search | Date of completion of the search | Examiner |
|---|---|---|
| Munich | 24 July 2023 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 23 38 2105

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/41765 A2 (SALVONA LLC [US]) 30 May 2002 (2002-05-30) * examples I-V * | 1,4,5,7, 9,11 | |
| X | WO 2018/054077 A1 (REYOUNG SUZHOU BIOLOGY SCIENCE & TECH CO LTD [CN]) 29 March 2018 (2018-03-29) | 1,4,5,7, 9,11,12, 15 | |
| Y | * examples 3-5; table 1 * | 8 | |
| A | | 13 | |
| X | SHARMA P ET AL: "Formulation and pharmacokinetics of lipid nanoparticles of a chemically sensitive nitrogen mustard derivative: Chlorambucil", INTERNATIONAL JOURNAL OF PHARMACEUTICS, ELSEVIER, NL, vol. 367, no. 1-2, 9 February 2009 (2009-02-09), pages 187-194, XP025879827, ISSN: 0378-5173, DOI: 10.1016/J.IJPHARM.2008.09.032 [retrieved on 2008-09-26] * table 1 * * page 188, left-hand column, paragraph materials, * | 1,2,4-7, 9,11 | |
| Y | IT MI20 080 516 A1 (PHARMAVAL SRL) 28 September 2009 (2009-09-28) * abstract; claims 1-10 * | 8 | |

**TECHNICAL FIELDS SEARCHED (IPC)**

The present search report has been drawn up for all claims

| Place of search | Date of completion of the search | Examiner |
|---|---|---|
| Munich | 24 July 2023 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 38 2105

This annex lists the patent family members relating to the patent documents cited in the above-mentioned European search report.
The members are as contained in the European Patent Office EDP file on
The European Patent Office is in no way liable for these particulars which are merely given for the purpose of information.

24-07-2023

| Patent document cited in search report | | Publication date | Patent family member(s) | | Publication date |
|---|---|---|---|---|---|
| WO 2022235238 | A2 | 10-11-2022 | TR 202107577 | A2 | 21-06-2021 |
| | | | WO 2022235238 | A2 | 10-11-2022 |
| WO 2011116963 | A2 | 29-09-2011 | BR 112012023638 | A2 | 03-10-2017 |
| | | | EP 2549977 | A2 | 30-01-2013 |
| | | | EP 3272398 | A1 | 24-01-2018 |
| | | | ES 2384060 | A1 | 29-06-2012 |
| | | | US 2013017239 | A1 | 17-01-2013 |
| | | | WO 2011116963 | A2 | 29-09-2011 |
| WO 0241765 | A2 | 30-05-2002 | AU 3977602 | A | 03-06-2002 |
| | | | EP 1328257 | A2 | 23-07-2003 |
| | | | US 6565873 | B1 | 20-05-2003 |
| | | | US 2003147956 | A1 | 07-08-2003 |
| | | | WO 0241765 | A2 | 30-05-2002 |
| WO 2018054077 | A1 | 29-03-2018 | CN 107865821 | A | 03-04-2018 |
| | | | EP 3515444 | A1 | 31-07-2019 |
| | | | JP 7018939 | B2 | 14-02-2022 |
| | | | JP 2019534870 | A | 05-12-2019 |
| | | | US 2019224194 | A1 | 25-07-2019 |
| | | | WO 2018054077 | A1 | 29-03-2018 |
| IT MI20080516 | A1 | 28-09-2009 | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HANTERA MM.** Trends in Dry Eye Disease Management Worldwide. *Clin Ophthalmol,* 2021, vol. 15, 173
- **LÓPEZ-MACHADO A ; DÍAZ-GARRIDO N ; CANO A ; ESPINA M ; BADIA J ; BALDOMÀ L et al.** Development of Lactoferrin-Loaded Liposomes for the Management of Dry Eye Disease and Ocular Inflammation. *Pharmaceutics,* 2021, vol. 13 (10), 1698
- **HUANG L ; GAO H ; WANG Z ; ZHONG Y ; HAO L ; DU Z.** Combination Nanotherapeutics for Dry Eye Disease Treatment in a Rabbit Model. *Int J Nanomedicine,* 2021, vol. 16, 3631
- **ALI F ; RAHUL ; NAZ F ; JYOTI S ; SIDDIQUE YH.** Health functionality of apigenin: A review. *Int J Food Prop,* 2016, vol. 20 (6), 1197-238
- *Clinical Trial to Evaluate the Reduction of Cardiovascular Risk - ClinicalTrials.gov n.d.,* 19 November 2021, https://clinicaltrials.gov/ct2/show/NCT04114916?term=apigenin&draw=4&rank=3
- **ZICK SM ; WRIGHT BD ; SEN A ; ARNEDT JT.** Preliminary examination of the efficacy and safety of a standardized chamomile extract for chronic primary insomnia: a randomized placebo-controlled pilot study. *BMC Complement Altern Med,* 2011, vol. 11, 78
- *Apigenbell 60 cápsulas Jellybell | Naturitas n.d.,* 19 November 2021, https://www.naturitas.es/p/suplementos/fitoterapia/compuestos-herbarios/apigenbell-60-capsulas-jelly-bell?gclid=CjwKCAiAs92MBhAXEiwAXTi25z_Q5qx2aeNYvzbrUCyGRG6PfdA91wTKXvtWHr461rXXYtFP_cDcZxoCkrAQAvD_BwE
- *Apigenina 90 cápsulas de 50mg Swanson | Naturitas n.d.,* 19 November 2021, https://www.naturitas.es/p/suplementos/suplementos-compuestos/apigenina-90-capsulas-de-50mg-swanson
- *Optrex Colirio para el Picor de Ojos n.d.,* 19 November 2021, https://www.optrex.es/gama-optrex/colirio/optrex-colirio-calmante-para-el-picor-de-ojos/optrex-colirio-calmante-para-el-picor-de-ojos
- *Optiben - Irritación ocular n.d.,* 19 November 2021, https://optiben.cmfa.com/Producto/7/alivio-natural-de-la-irritacin-ocular

- **WANG M ; FIRRMAN J ; LIU LS ; YAM K.** A review on flavonoid apigenin: Dietary intake, ADME, antimicrobial effects, and interactions with human gut microbiota. *Biomed Res Int,* 2019, 7010467
- **BATTAGLIA L ; SERPE L ; FOGLIETTA F ; MUNTONI E ; GALLARATE M ; DEL POZO RODRIGUEZ A et al.** Application of lipid nanoparticles to ocular drug delivery. *Expert Opin Drug Deliv,* 2016, vol. 13 (12), 1743-57
- **FANGUEIRO JF ; CALPENA AC ; CLARES B ; ANDREANI T ; EGEA MA ; VEIGA FJ et al.** Biopharmaceutical evaluation of epigallocatechin gallate-loaded cationic lipid nanoparticles (EGCG-LNs): In vivo, in vitro and ex vivo studies. *Int J Pharm,* 2016, vol. 502 (1-2), 161-9
- **AGARWAL P ; CRAIG JP ; RUPENTHAL ID.** Formulation Considerations for the Management of Dry Eye Disease. *Pharmaceutics,* 2021, vol. 13 (2), 207
- **CARVAJAL-VIDAL P ; FÁBREGA MJ ; ESPINA M ; CALPENA AC ; GARCIA ML.** Development of Halobetasol-loaded nanostructured lipid carrier for dermal administration: Optimization, physicochemical and biopharmaceutical behavior, and therapeutic efficacy. *Nanomedicine Nanotechnology, Biol Med,* 2019, vol. 20, 102026
- **SÁNCHEZ-LÓPEZ E ; ETTCHETO M ; EGEA MA ; ESPINA M ; CANO A ; CALPENA AC et al.** Memantine loaded PLGA PEGylated nanoparticles for Alzheimer's disease: In vitro and in vivo characterization. *J Nanobiotechnology,* 2018, vol. 16 (1), 1-16
- **SÁNCHEZ-LÓPEZ E ; ESTERUELAS G ; ORTIZ A ; ESPINA M ; PRAT J ; MUÑOZ M et al.** Dexibuprofen Biodegradable Nanoparticles: One Step Closer towards a Better Ocular Interaction Study. *Nanomaterials,* 2020, vol. 10 (4
- **ZEB A ; QURESHI OS ; KIM HS ; KIM MS ; KANG JH ; PARK JS et al.** High payload itraconazole-incorporated lipid nanoparticles with modulated release property for oral and parenteral administration. *J Pharm Pharmacol,* 2017, vol. 69 (8), 955-66
- **SOUTO EB ; MEHNERT W ; MÜLLER RH.** Polymorphic behaviour of Compritol888 ATO as bulk lipid and as SLN and NLC. *J Microencapsul,* 2006, vol. 23 (4), 417-33
- **FREITAS C ; MÜLLER RH.** Correlation between long-term stability of solid lipid nanoparticles (SLN) and crystallinity of the lipid phase. *Eur J Pharm Biopharm,* 1999, vol. 47 (2), 125-32

- **ZIMMERMANN E ; SOUTO EB ; MÜLLER RH.** Physicochemical investigations on the structure of drug-free and drug-loaded solid lipid nanoparticles (SLN) by means of DSC and 1H NMR. *Pharmazie,* 2005, vol. 60 (7), 508-13

- **ALSHEHRI SM ; SHAKEEL F ; IBRAHIM MA ; EL-ZAYAT EM ; ALTAMIMI M ; MOHSIN K et al.** Dissolution and bioavailability improvement of bioactive apigenin using solid dispersions prepared by different techniques. *Saudi Pharm J,* 2019, vol. 27 (2), 264-73

- *tability of Dispersions | 3P Instruments n.d.,* 22 November 2021, https://www.3p-instruments.com/measurement-methods/stability-turbiscan

- **LIU P ; DE WULF O ; LARU J ; HEIKKILÄ T ; VAN VEEN B ; KIESVAARA J et al.** Dissolution Studies of Poorly Soluble Drug Nanosuspensions in Non-sink Conditions. *AAPS PharmSciTech,* 2013, vol. 14 (2), 748

- **HUANG D ; CHEN YS ; RUPENTHAL ID.** Hyaluronic Acid Coated Albumin Nanoparticles for Targeted Peptide Delivery to the Retina. *Mol Pharm,* 2017, vol. 14 (2), 533-45

- **LAGARTO A ; VEGA R ; GUERRA I ; GONZÁLEZ R.** In vitro quantitative determination of ophthalmic irritancy by the chorioallantoic membrane test with trypan blue staining as alternative to eye irritation test. *Toxicol In Vitro,* 2006, vol. 20 (5), 699-702

- **XIONG C ; CHEN D ; LIU J ; LIU B ; LI N ; ZHOU Y et al.** A rabbit dry eye model induced by topical medication of a preservative benzalkonium chloride. *Invest Ophthalmol Vis Sci,* 2008, vol. 49 (5), 1850-6

- **HOLZCHUH R ; VILLA ALBERS MB ; OSAKI TH ; IGAMI TZ ; SANTO RM ; KARA-JOSE N et al.** Two-year outcome of partial lacrimal punctal occlusion in the management of dry eye related to Sjögren syndrome. *Curr Eye Res,* 2011, vol. 36 (6), 507-12

- **LIN TK ; ZHONG L ; SANTIAGO JL.** Anti-Inflammatory and Skin Barrier Repair Effects of Topical Application of Some Plant Oils. *Int J Mol Sci,* 2017, vol. 19 (1), 70

- **KIRALAN M ; YILDIRIM G.** Rosehip (Rosa canina L.) Oil. *Fruit Oils Chem Funct,* 2019, 803-14